# EUROPEAN PATENT SPECIFICATION

(11) **EP 2 663 244 B1**
(45) Date of publication and mention of the grant of the patent: **20.09.2023**
(21) Application number: 12734636.9
(22) Date of filing: 11.01.2012
(51) Int. Cl.: A61B 17/12, A61B 17/064, A61B 17/00

(54) **APPARATUS FOR SECURING TISSUE LAYERS TOGETHER**
VORRICHTUNG ZUM BEFESTIGEN VON GEWEBESCHICHTEN ANEINANDER
APPAREIL POUR FIXER DES COUCHES DE TISSU ENSEMBLE

(30) Priority: 11.01.2011 US 201161431609 P
(43) Date of publication of application: 20.11.2013
(73) Proprietor: Amsel Medical Corporation, Cambridge, MA 02139 (US)
(72) Inventor: MILLER, Arnold, Cambridge, MA 02139 (US); LORE, Avraham, Rami, 36082 Kiryat Tivon (IL)
(74) Representative: Mewburn Ellis LLP
(86) International application number: PCT/US2012/020973
(87) International publication number: WO 2012/097086

(56) References cited:
- WO-A1-2005/110240
- WO-A2-02/17771
- WO-A2-2007/002307
- WO-A2-2010/127083
- JP-A- 2009 291 476
- US-A- 4 800 879
- US-A- 5 334 217
- US-A1- 2004 138 684
- US-A1- 2005 277 966
- US-A1- 2006 264 987
- US-A1- 2007 179 527
- US-A1- 2008 306 495
- US-A1- 2009 084 386
- US-A1- 2009 125 038
- US-B1- 7 798 953
- US-B1- 8 133 242

## Description

### Field Of The Invention

This invention relates to surgical apparatus for the occlusion of blood vessels and the treatment of varicose veins by securing tissue layers together.

### Background Of The Invention

### Varicose Veins In General

There are three sets of veins in the legs:
(i) superficial veins that lie under the skin and may be seen and felt when standing; (ii) deep veins that lie within the muscles and are not seen or felt; and (iii) perforating or connecting veins that join the two systems (i.e., the superficial veins and the deep veins).

Veins lie within all tissues. Veins return blood to the heart. When muscles in the leg contract, blood is pumped back to the heart. Valves inside the veins direct the flow of blood back to the heart.

The veins are relatively weak tubes. Under the skin there is no support for these veins, so that when the pressure in the veins is elevated, areas of weakness occur and the veins enlarge, both in size and length. In some cases the veins can become twisty and bulge significantly. This condition is commonly referred to as varicose veins.

Very small varicose veins are sometimes called spider veins. Unlike the larger varicose veins, these spider veins lie in the skin.

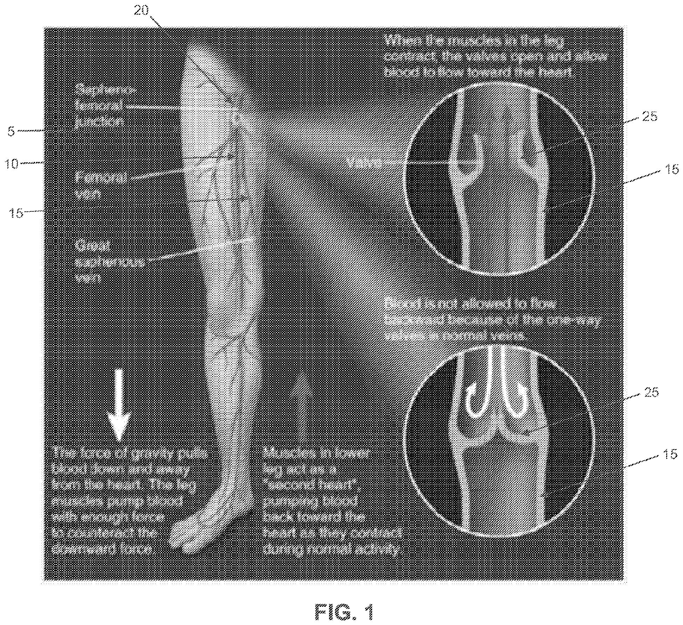

The cause of the increased pressure in the veins is due to the occurrence of "leaky" valves within the veins. The main valve is in the groin region, i.e., in the great sapheous vein near the sapheno-femoral junction. See Fig. 1, which shows a leg 5 of a patient, the femoral vein 10, the great saphenous vein 15, the sapheno-femoral junction 20, and the main valve 25 in the great saphenous vein near the sapheno-femoral junction. Once this main valve in the saphenous vein becomes leaky, the pressure in the vein increases and the veins below the saphenous vein start to enlarge. This causes the next set of valves in the saphenous vein to leak. The raised pressure caused by the leaky valves in the saphenous vein is transmitted to the feeder veins, which distend and their valves also malfunction and become leaky. As this process carries on down the leg, many of the valves in the leg veins become incompetent, with high pressures occurring in the veins, especially on standing.

Initially, the problem is primarily cosmetic. The veins bulge and look unsightly. However, there is commonly also discomfort in the legs upon standing. This discomfort is the result of the veins distending due to the increased pressure.

With time, the high pressure in the veins is transmitted to the surrounding tissues and skin. Small veins within the skin (i.e., spider veins) enlarge and become visible. Blood cells may escape into the tissues and break down, causing areas of discoloration. Because the pressure in the tissues is high, the skin swells and the nutrition of the skin deteriorates. This lowers the local tissue resistance and allows infection to occur. Eventually skin may break down with the development of sores (i.e., ulcers).

### Incidence Of Varicose Veins

Nearly 40 percent of women and 25 percent of men suffer from lower extremity venous insufficiency and associated visible varicose veins. Primary risk factors include heredity, gender, pregnancy and age. Most of these patients have long-standing leg symptoms which compromise their daily routine, with symptoms worsening during the day while the patients are at work or simply living their lives. Without varicose vein treatment, these symptoms can progress to a lifestyle-limiting condition.

### Treatment Of Varicose Veins

Treatment of varicose veins is undertaken for relief of the symptoms, i.e., the removal of the unsightly veins and the prevention of the discomfort and late-stage manifestations described above.

### 1. Non-Surgical Treatment.

The simplest treatment is a non-surgical treatment directed against the high pressure in the varicose veins. More particularly, fitted elastic stockings, strong enough to overcome the increased pressure caused by the "leaky" valves, are used. These fitted elastic stockings control the symptoms and may prevent the veins from further enlargement, however, they are not curative. Good results require consistent, every-day use of the stockings.

### 2. Surgical/Interventional Treatment.

The aim of the surgical/interventional treatment is (i) the elimination of the cause of the high venous pressure (i.e., the "leaky" valves at the groin); and (ii) the removal of the unsightly veins.

The early approach of "stripping" the saphenous vein (the main vein in the leg) as the sole manner of treatment has now been largely abandoned. This is because the "stripping" approach caused too much trauma and did not remove all of the superficial varicose veins: many of the superficial varicose veins were tributaries of the main superficial vein of the leg (i.e., the saphenous vein) that was stripped, and these tributary veins were not removed by this procedure.

There are currently three basic approaches for treating varicose veins: chemical - sclerorosants and glues; venous ablation using thermal treatments; and open surgery.

### A. Sclerotherapy.

Sclerotherapy (the use of sclerosants) is generally used for treating the smaller varicose veins and spider veins that do not appear to be directly associated with "leaky" valves. It is primarily a cosmetic procedure.

In this approach, a sclerosant (i.e., a substance irritating to the tissues) is injected into the smaller varicose veins and spider veins, causing inflammation of the walls of these veins. As a result of this inflammation, the walls of the vein stick together and occlude the lumen of the vein so that no blood can pass through the vein. Eventually these veins shrink and disappear.

The disadvantages of sclerotherapy include:
(i) in the presence of high venous pressure (i.e., with leaky valves and the larger varicose veins), the results are uncertain and the recurrence rate is high; and (ii) the erroneous injection of the sclerosant into the surrounding tissues can result in damage to the surrounding tissues, with areas of discoloration of the skin and even ulceration.

Recently, mixing the sclerosant with air to form a "foam" has been used to destroy the lining of the main vein (i.e., the saphenous vein) of the leg. To date, the results are somewhat unpredictable and there is a danger of the sclerosant escaping through the saphenous vein and into the deep veins and then embolizing into the lungs, which is harmful and dangerous for the patient.

### B. Venous Ablation.

Venous ablation for varicose veins can be effected in two ways, i.e. percutaneously and endovenously.

With the percutaneous approach, the superficial smaller varicose veins and spider veins are "heated" and coagulated by shining an external laser light through the skin. However, if the veins are too large, the amount of energy needed to destroy the veins may result in damage to the surrounding tissues. Percutaneous laser treatment is primarily an alternative to the sclerotherapy discussed above, and generally suffers from the same disadvantages described above with respect to sclerotherapy.

With endovenous ablation, a special laser or radio-frequency (RF) catheter is introduced, with local anesthesia, through a needle puncture into the main superficial vein (i.e., the saphenous vein) of the leg. Entry is made in the region around the knee, and the catheter is passed up towards the groin, advancing to the site where the saphenous vein joins the deep veins at the site of the main "leaky" valves. Then, as the catheter is slowly withdrawn back through the vein, the laser light or radio-frequency (RF) energy heats up the wall of the vein, endoluminally coagulating the proteins and destroying the lining surface of the vein. The destruction of the lining surface of the vein causes the vein walls to adhere to one another, thereby eliminating the lumen within the vein and thus preventing the flow of blood. This is a process somewhat similar to sclerotherapy, but no substance is injected into the vein. This procedure takes care of the "leaky" valves and high venous pressures, however, the larger superficial varicose veins in the leg may still need to be removed. This may be done at the same time as the endovenous ablation or at a later time, either by open surgery (phlebectomy) or sclerotherapy. Placement of the laser or radio-frequency (RF) catheter is guided by ultrasound.

The advantages of endovenous laser/radio-frequency (RF) therapy include: (i) it is a minimally invasive procedure and can be done with local anesthesia, either in an operating room or a physician's office; (ii) it does not require hospitalization; (iii) it does not require open surgery with incisions; (iv) recovery is easier than with open surgery, inasmuch as most patients are back at work within a day or two; and (v) some of the prominent varicosities may disappear and may not require a secondary procedure (i.e., either phlebectomy or sclerotherapy).

The disadvantages of endovenous laser/radio-frequency (RF) therapy include: (i) generally, only one leg is done at a time; (ii) the procedure typically requires significant volumes of local anesthetic to be injected into the patient in order to prevent the complications of the heat necessary to destroy the lining of the vein; (iii) if too much heat is applied to the tissue, there can be burning in the overlying skin, with possible disfiguring, including scarring; (iv) prior to the performance of a subsequent phlebectomy procedure, an interval of up to 8 weeks is required in order to evaluate the effectiveness of the venous ablation procedure; and (v) varicosities that remain after this interval procedure still require separate procedures (i.e., phlebectomy or sclerothapy).

### C. Open Surgery.

The aim of open surgery is to eliminate the "leaky" valve at the junction of the superficial and deep veins (the cause of the high venous pressure in the leg), as well as the leaky valves in the tributaries of the saphenous vein that may enlarge over the years and result in a recurrence of the varicose veins. This open surgery is directed to removal of some or all of the affected veins.

There is still some controversy as to how much of the saphenous vein needs to be removed for the best results. The current "teaching" is that removing the entire segment of saphenous vein in the thigh reduces the incidence of recurrence. However, the data for this is very weak. Removal of a very short segment of the proximal saphenous vein and the main tributaries at the sapheno-femoral junction is the alternative procedure and, provided that it is combined with removal of all visible varicosities, the results are very similar to removal of the entire thigh segment of the saphenous vein. The advantage of the latter procedure is the increased preservation of the saphenous vein which, in 50-60% or more of varicose vein patients, is not involved in the varicose vein process and is otherwise normal and hence usable for other procedures (such as a bypass graft in the heart or limbs).

The surgery is performed in the operating room under light general or regional (spinal or epidural) anesthesia. An incision (e.g., 1-2 inch) is made in the groin crease and the veins dissected out and the proximal saphenous vein and tributaries excised. The wound is closed with absorbable sutures from within. Once this is completed, small (e.g., 2-4 mm) stab wounds are made over any unsightly varicose veins (these veins are marked out just prior to the surgery with the patient standing) and the varicose veins are completely removed. The small stab wounds associated with removal of the marked-out veins are generally so small that they typically do not require any stitches to close them. When all the previously marked-out veins are removed, the wounds are cleaned and a dressing applied. The leg is wrapped in elastic bandages (e.g., Ace wraps).

In the post-operative care, the dressings and Ace wraps are usually changed in the doctor's office at the first post-operative visit, typically within 24 hours of the open surgical procedure. The patient and a family member or friend is instructed on proper care of the wounds. A simple dressing is applied to cover the small wounds in the legs for the next 2-3 days. After 2-3 days no further treatment is generally required. Recovery is generally rapid, with the patient returning to work within 5-7 days.

The advantages of open surgery include:
(i) varicose veins of both extremities can be done at a single operation, which generally takes 1-2 hours; (ii) the procedure typically does not require hospitalization and is an "out patient" procedure; (iii) the wounds are minimal, with minimal discomfort which is easily managed with oral analgesics (i.e., pain medicine); (iv) the results are generally excellent, with a minimum of recurrence (the results of open surgery remain the "gold standard" against which the sclerotherapy and laser/radio-frequency (RF) venous ablation therapies are compared); (v) recurrent or residual (i.e., those missed at surgery) veins are generally managed with sclerotherapy or phlebectomy under local anesthesia in a doctor's office or in an ambulatory procedure room; and (vi) the saphenous vein, if normal and without varicosities, is preserved and is therefore available for use (e.g., for bypass surgery) in the future if it should be needed.

The disadvantages of open surgery include:
(i) it is an open surgical procedure requiring an anesthetic (either general or regional), with its associated discomfort and with its attendant risks (which may depend on the health or age of the patient); and (ii) recovery generally takes 3-5 days.

Thus it will be seen that varicose veins present a significant problem for many patients which must be addressed, and all of the current procedures for treating varicose veins suffer from a number of significant disadvantages.

### Further Background of Art

WO 02/17771 discloses a surgical fastener, which can access internal tissue or other synthetic material by catheter delivery through an endovascular pathway. The surgical fastener uses superelastic properties of a shape memory alloy and so being able to access internal tissue or other synthetic material through a small surgical access port or incision

However, this state of art does not overcome before mentioned deficiencies.

US 2005/0277966 A1 discloses an apparatus and methods for optimizing anchoring force. In securing tissue folds, over-compression of the tissue directly underlying the anchors is avoided by utilizing tissue anchors having expandable arms configured to minimize contact area between the anchor and tissue.

US 2009/0125038 A1 discloses a ligature delivery device including a control member, an elongated shaft, and an end effector attached to the distal end of the elongated shaft. An activation mechanism provides an user-operable connection between the control member and the end effector. In several embodiments, the end effector includes a reverse grasping mechanism. Several embodiments of ligature devices are adapted to be deployed endoscopically and/or translumenally using the reverse-grasping delivery device.

### Summary Of The Invention

The present invention is as defined in the appended set of claims.

### Brief Description Of The Drawings

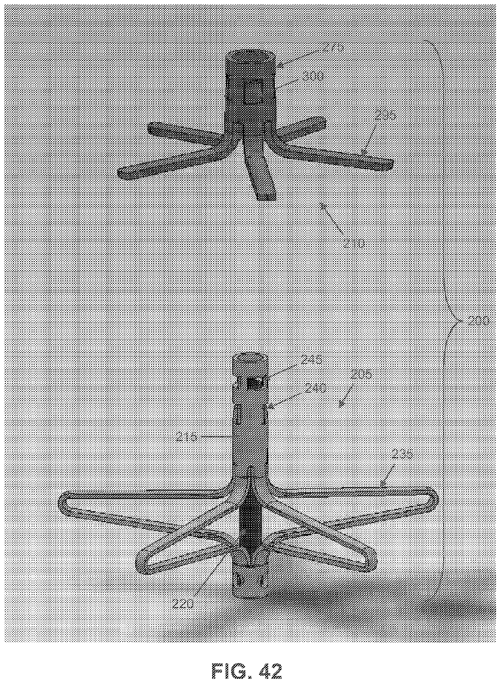

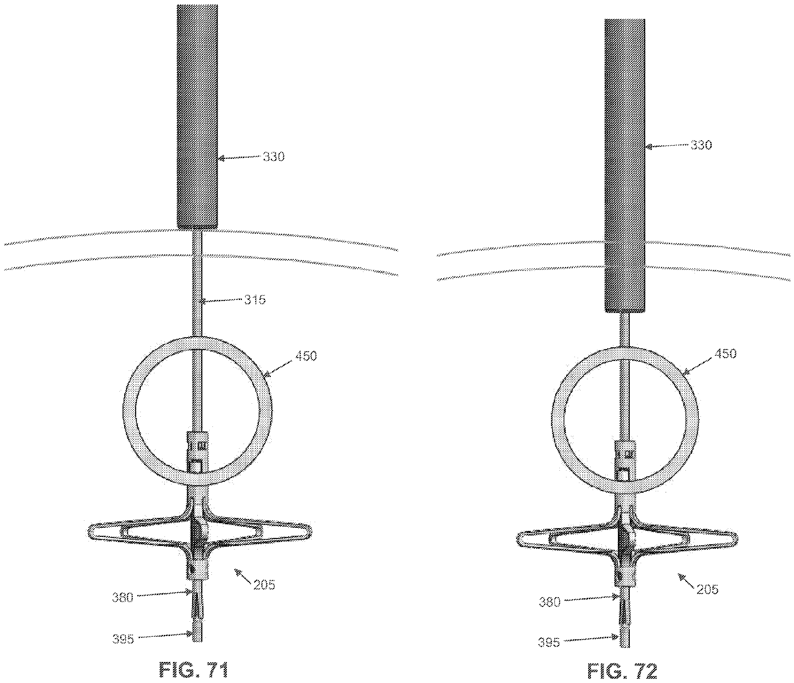

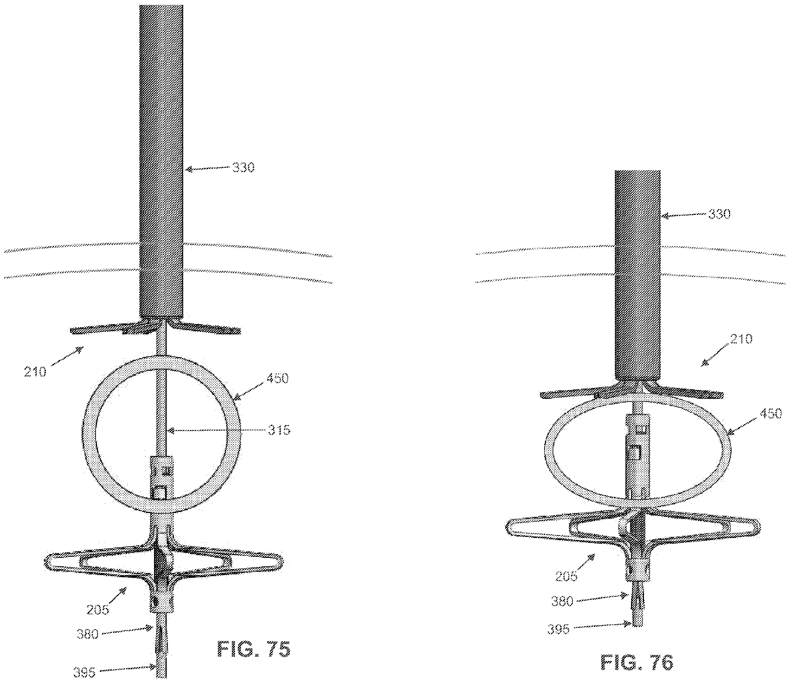

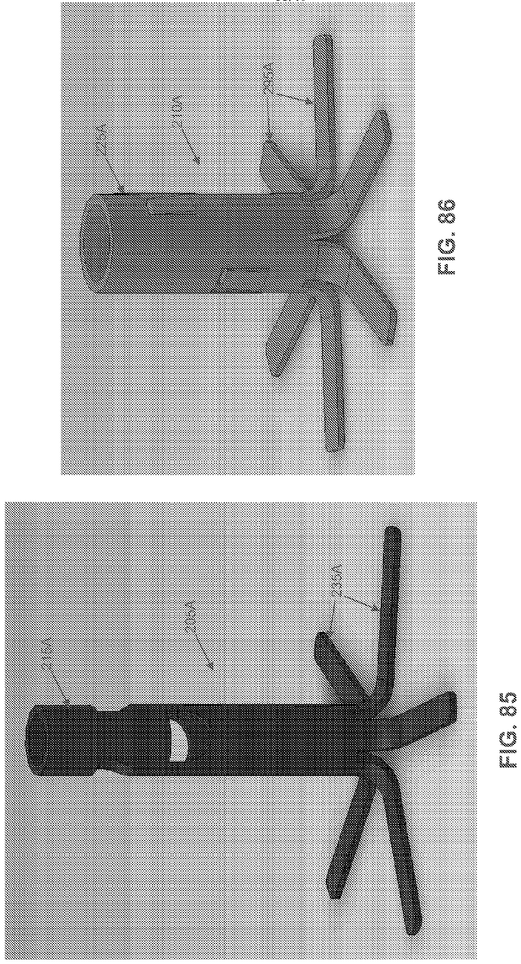

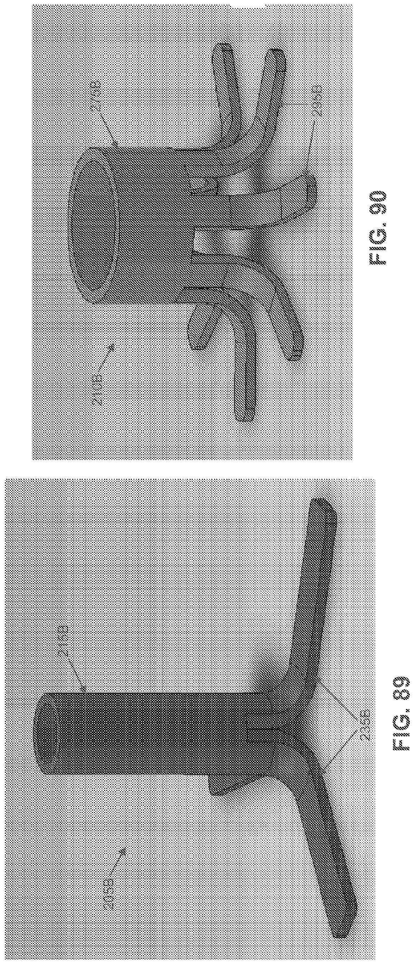

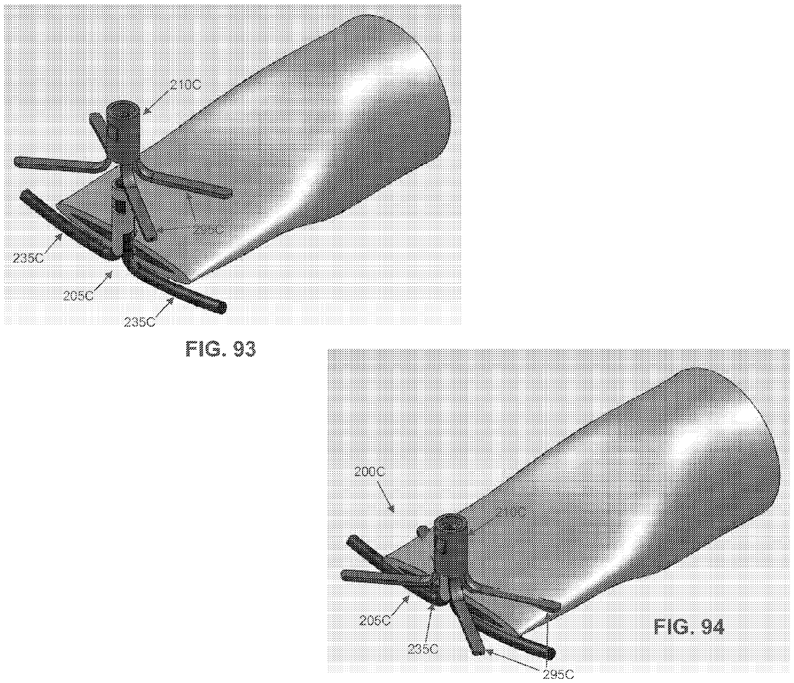

The objects and features of the present invention will be more fully disclosed or rendered obvious by the following detailed description of the preferred embodiments of the invention, which is to be considered together with the accompanying drawings wherein figures 42-94 illustrate embodiments of the present invention, wherein like numbers refer to like parts, and further wherein:
Fig. 1 is a schematic view showing various aspects of the venous system of the leg;
Figs. 2-4 are schematic views showing an occluder occluding a blood vessel not in accordance with the present invention;
Fig. 5 is a schematic view showing one possible construction for the occluder shown in Figs. 2-4;
Figs. 6 and 7 are schematic views showing an exemplary syringe-type inserter which may be used to deploy the occluder shown in Figs. 2-4;
Figs. 8-10 are schematic views showing an occluder occluding a blood vessel not in accordance with the present invention;
Figs. 11-14 are schematic views showing an occluder occluding a blood vessel not in accordance with the present invention;
Figs. 15-17 are schematic views showing other possible constructions for the occluder not in accordance with the present invention;
Figs. 18-20 are schematic views showing the occluders of the types shown in Figs. 15-17 occluding a blood vessel not in accordance with the present invention;
Figs. 21-24 are schematic views showing an occluder occluding a blood vessel not in accordance with the present invention;
Figs. 25-27 are schematic views showing an occluder occluding a blood vessel not in accordance with the present invention;
Figs. 28 and 29 are schematic views showing an occluder occluding a blood vessel not in accordance with the present invention;
Figs. 30 and 31 are schematic views showing an occluder occluding a blood vessel not in accordance with the present invention;
Figs. 32 and 33 are schematic views showing an occluder occluding a blood vessel not in accordance with the present invention;
Figs. 34 and 35 are schematic views showing a drug/cellular delivery body being attached to a blood vessel not in accordance with the present invention;
Figs. 36 and 37 are schematic views showing a drug/cellular delivery body being attached to a blood vessel not in accordance with the present invention;
Figs. 38 and 39 are schematic views showing a drug/cellular delivery body being attached to a blood vessel not accordance with the present invention;
Figs. 40 and 41 are schematic views showing a drug/cellular delivery body being attached to a blood vessel not in accordance with the present invention;
Figs. 42-48 are schematic views showing a two-part occluder formed in accordance with the present invention;
Figs. 49-58 are schematic views showing installation apparatus which may be used to deploy the two-part occluder of Figs. 42-48;
Figs. 59-82 are schematic views showing the two-part occluder of Figs. 42-48 being deployed across a blood vessel using the installation apparatus of Figs. 49-58;
Figs. 83-86 are schematic views showing another two-part occluder formed in accordance with the present invention;
Figs. 87-90 are schematic views showing still another two-part occluder formed in accordance with the present invention;
Figs. 91-94 are schematic views showing yet another two-part occluder formed in accordance with the present invention; and
Figs. 95-100 are schematic views showing another two-part occluder, however not formed in accordance with the present invention.

### Detailed Description

The present description provides various approaches for treating varicose veins and other blood vessels.

More particularly, the present description comprises the provision and use of occluders used to occlude a vein (e.g., the proximal saphenous vein, the small saphenous vein, tributaries, the perforator veins, etc.) so as to restrict blood flow through the vein and thereby treat varicose veins below the point of occlusion. Significantly, the described occluders are configured to be deployed using a minimally-invasive approach (i.e., either percutaneously or endoluminally), with visualization being provided by ultrasound and/or other visualization apparatus (e.g., CT, MRI, X-ray etc.). As a result, the described treatment can be provided in a doctor's office, with minimal local anesthetic, and effectively no post-operative care.

### Percutaneous Approach

In the percutaneous approach, the occluder is delivered by percutaneously advancing the occluder through the skin, through intervening tissue and then across some or all of the blood vessel (e.g., the great saphenous vein near the sapheno-femoral junction) so as to occlude the blood vessel. This occlusion (or multiple of these occlusions) will thereby treat varicose veins. In one form of the invention, the occluder is configured to occlude the vein by compressing the vein and closing down its lumen; and in another form not of the invention, the occluder is configured to occlude the vein by depositing a mass within the lumen of the vein so as restrict blood flow through the lumen of the vein. The occlusion of the lumen may be complete or partial. If the occlusion is partial, some blood may continue to flow in the vein. Such partial occlusion can act to relieve some of the pressure on the valve, thereby improving its function. In some applications, an occlusion of 70% or greater of the lumen may be desired and realized based on the current invention. In other applications, an occlusion of 80% or greater of the lumen may be desired and realized based on the current invention. In one embodiment, the occlusion pressure applied may be greater than 40 mm of mercury. In another embodiment of the present invention, the occlusion pressure may be greater than the pressure of the typical blood flow in the vein.

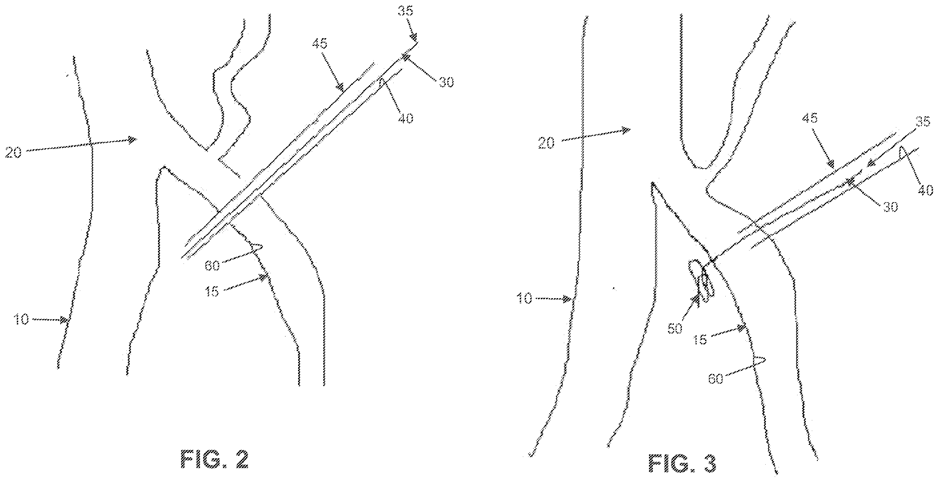

Looking first at Figs. 2-4, in one form not according to the invention, there is provided an occluder 30. Occluder 30 comprises an elastic filament 35 which, in an unconstrained condition, comprises a generally non-linear configuration (e.g., a coiled mass) but which, when properly restrained, can maintain a linear configuration (e.g., in the narrow lumen 40 of a needle 45, or where the filament is formed out of a shape memory material, by appropriately controlling its temperature and hence its shape); when the restraint is removed (e.g., the elastic filament 35 is extruded from the constraining lumen 40 of the needle 45, or the temperature of the shape memory material is elevated such as by body heat), elastic filament 35 will return to its generally non-linear configuration, whereby to provide enlarged masses for occluding the vein.

In one form, the occluder is formed out of a shape memory material (e.g., a shape memory alloy such as Nitinol, or a shape memory polymer), with the shape memory material being configured to provide superelasticity, or temperature-induced shape changes, or both).

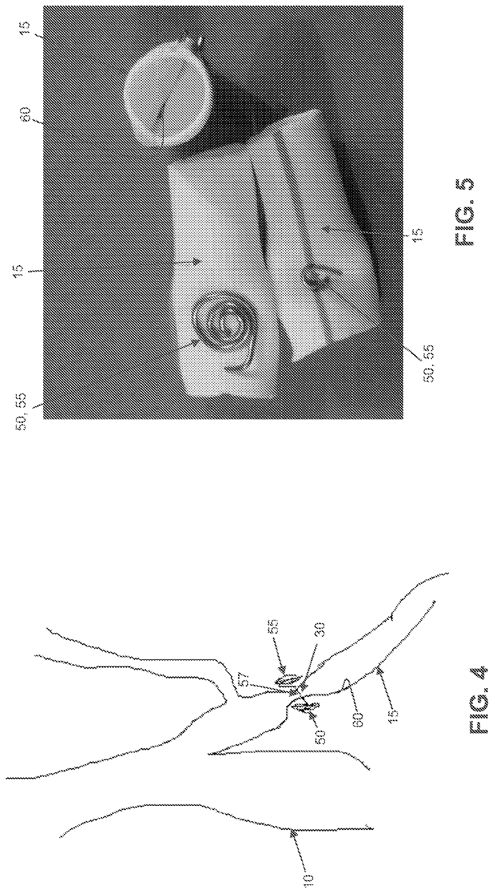

In one preferred method of use, the occluder 30 is installed in the narrow lumen 40 of a needle 45 (Fig. 2), the needle is introduced percutaneously and advanced across the vein which is to be occluded (e.g., the great saphenous vein 15), a first length of the occluder is extruded from the needle on the far side of the vein so that a portion of the occluder is restored to a coiled mass configuration 50 on the far side of the vein (Fig. 3), the needle is withdrawn back across the vein, and then the remainder of the occluder is extruded on the near side of the vein (Fig. 4), whereupon the remainder of the occluder is restored to a coiled mass configuration 55, with a portion 57 of the occluder extending across the lumen 60 of the vein 15, and with the portions of the occluder on the far and near sides of the vein (i.e., the coiled masses 50 and 55, respectively) being drawn toward one another under the coiling force inherent in the elastic filament so as to compress the vein there between and occlude its lumen 60, whereby to restrict blood flow through the vein and thereby treat the varicose veins.

As noted above, occluder 30 may be formed out of a shape memory material (e.g., a shape memory alloy such as Nitinol, or a shape memory polymer, etc.), with the shape memory material being configured to provide superelasticity, or temperature-induced shape changes, or both).

In the form shown in Figs. 2-4, occluder 30 is formed out of a single elastic filament 35, and a shape transition (i.e., from substantially linear to a pair of opposing coiled masses 50, 55) is used to cause occlusion of the target blood vessel. In this respect it should be appreciated that the aforementioned coiled masses 50, 55 may comprise substantially random turns of the elastic filament arranged in a substantially three-dimensional structure (i.e., somewhat analogous to a ball of string), or the coiled masses 50, 55 may comprise highly reproducible structures such as loops, coils, etc., and these loops, coils, etc. may or may not assume a substantially planar structure. See, for example, Fig. 5, where coiled masses 50, 55 comprise highly reproducible loops and coils.

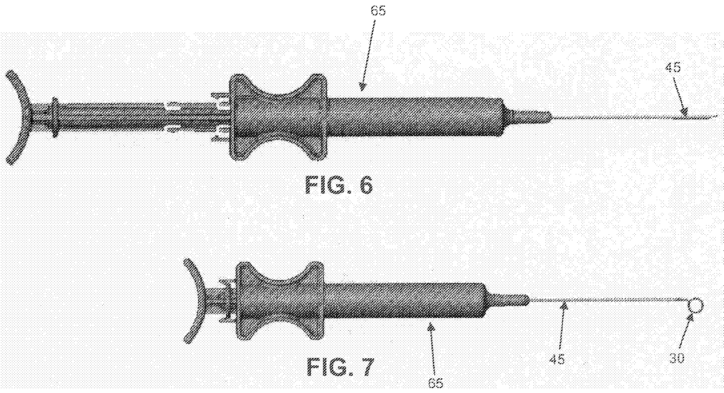

Figs. 6 and 7 show an exemplary syringe-type inserter 65 which may be used to deploy the occluder. The syringe-type inserter 65 may contain one occluder 30 or multiple pre-loaded occluders 30, e.g., where syringe-type inserter 65 comprises multiple occluders 30, the occluders may be disposed serially within the syringe-type inserter, or they may be disposed parallel to one another within the syringe-type inserter (i.e., in the manner of a "Gatling gun" disposition), etc. When the syringe-type inserter 65 is activated, an occluder 30 is deployed out of the distal end of needle 45.

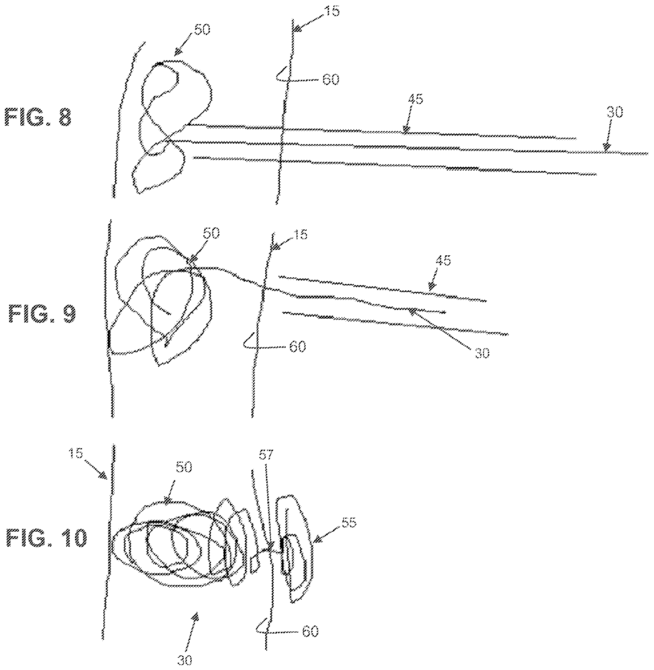

In Figs. 2-4, occluder 30 is shown occluding the vein by compressing the vein between the two coiled masses 50, 55, whereby to close down its lumen 60. However, in another form of the invention, the occluder 30 can be used to occlude the vein without compressing the vein. This is done by depositing a coiled mass within the lumen of the vein, whereby to restrict blood flow through the lumen of the vein. More particularly, and looking now at Figs. 8-10, the needle 45 is passed into the interior of the vein 15 and one coiled mass 50 of the occluder 30 is extruded into the lumen 60 of the vein (Fig. 8) so as to occlude the lumen of the vein, the needle 45 is withdrawn to the near side of the vein (Fig. 9), and then another coiled mass 55 is disposed on the near side of the vein (Fig. 10), with the portion 57 of the occluder extending through the side wall of the vein so as to stabilize the occluder relative to the vein (i.e., so as to attach the occluder to the vein and prevent the occluder from moving relative to the vein).

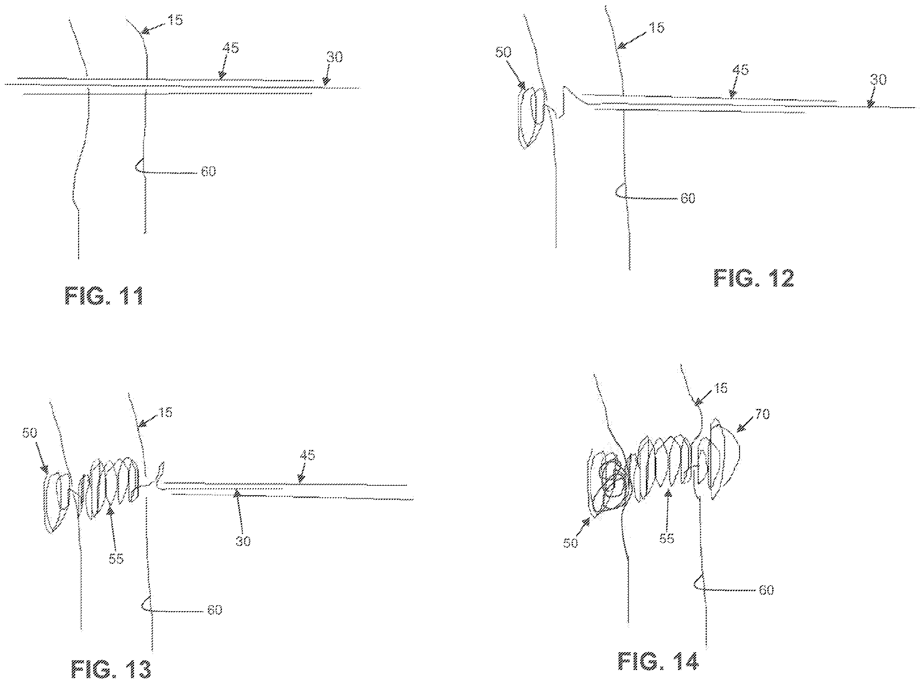

Figs. 11-14 show another approach where a coiled mass of the occluder 30 is deposited within the interior of the blood vessel so as to obstruct blood flow through the vessel. More particularly, the needle 45 is passed completely through the vein (Fig. 11), a coiled mass 50 of the occluder is deposited on the far side of the vein (Fig. 12), the needle is withdrawn into the interior of the vein where another coiled mass 55 of the occluder is deposited (Fig. 13), and then the needle is withdrawn to the near side of the vein where another coiled mass 70 of the occluder 30 is deposited (Fig. 14). Coiled mass 55 resides within the lumen 60 of the vein and obstructs blood flow while coiled masses 50 and 70 compress the vein inwardly and stabilize the disposition of the intraluminal coiled mass 55.

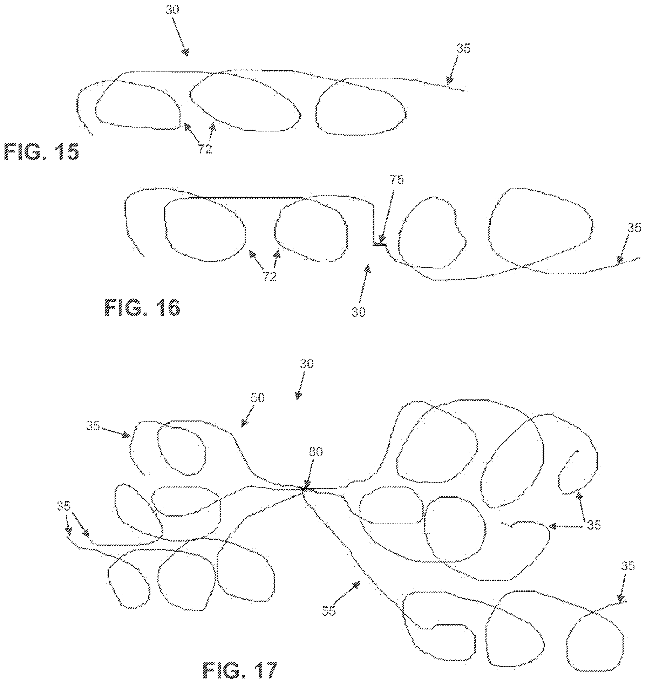

Figs. 15 and 16 show occluders 30 formed out of a single strand of elastic filament. In Fig. 15, the occluder 30 comprises a relatively ordered coil where the turns 72 of the coil are unidirectional. In Fig. 16, the occluder 30 comprises another relatively ordered coil but where the turns rotate in opposite directions on different sides of a midpoint 75. Of course, it should also be appreciated that the occluder 30 can be constructed so as to form a relatively disordered coil, i.e., where the strand of the filament follows a relatively random pattern (see, for example, the disordered coils illustrated in Figs. 8-10). Indeed, where it is desired that the mass of the reformed coil itself provide a flow obstruction (e.g., where the reformed coil is disposed intraluminally so as to impede blood flow through the vein), it is generally preferred that the elastic filament reform into a relatively disordered coil having a relatively random disposition, since this can provide a denser filament configuration.

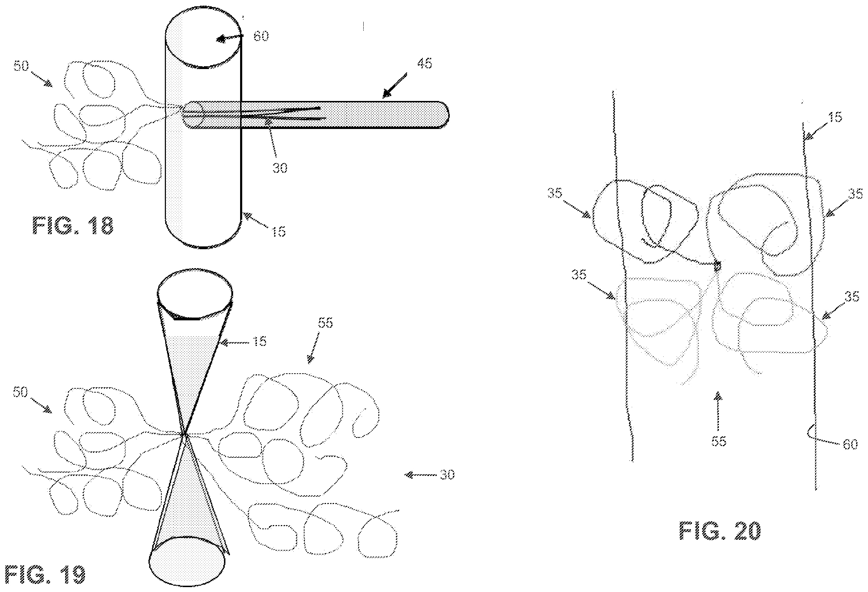

Fig. 17 shows an occluder 30 formed out of multiple strands of elastic filaments 35. These multiple strands are joined together at a joinder 80. Again, the coils (e.g., the aforementioned coiled masses 50, 55, 70) formed by these multiple strands can be relatively ordered or relatively disordered. Figs. 18 and 19 show how the multistrand occluder of Fig. 17 can be used to occlude a vein by forming coiled masses 50, 55 to compress the side wall of the vein inwardly so as to restrict blood flow through the vein. Fig. 20 shows how the multistrand occluder 30 of Fig. 17 can be used to occlude a vein by depositing a coiled mass 55 within the lumen 60 of the vein, whereby to restrict blood flow through the lumen of the vein. In Fig. 20, a number of the elastic filaments 35 are shown piercing the side wall of the vein so as to hold the coiled mass 55 in position within the lumen of the blood vessel.

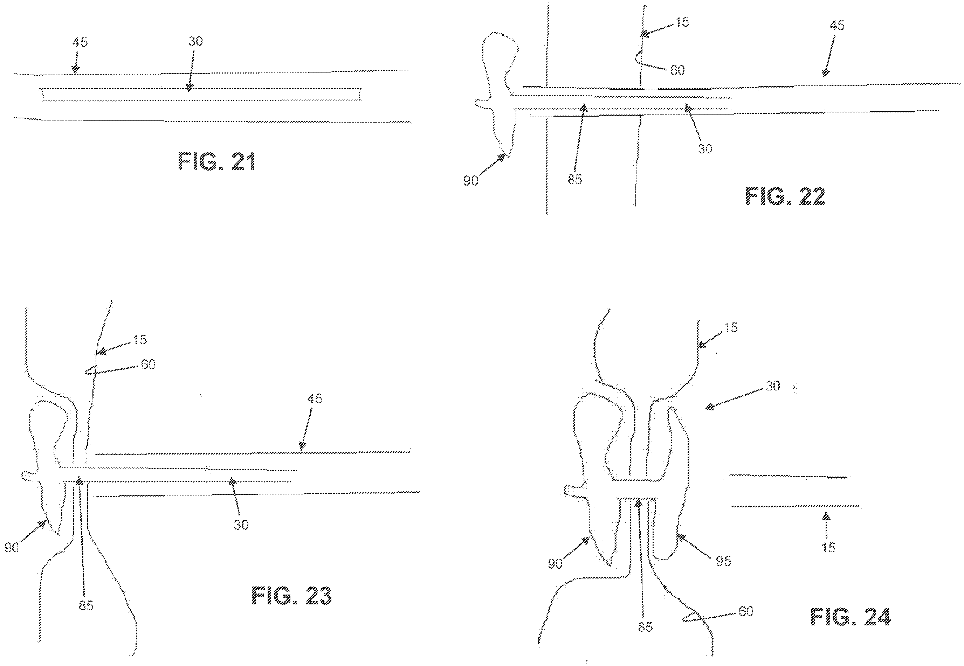

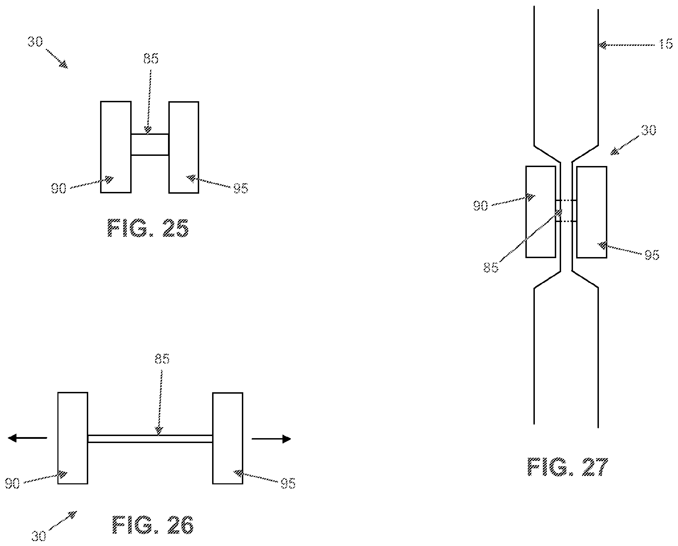

Figs. 21-24 show another form of occluder 30 where the occluder is formed by structures other than a filament. By way of example, the occluder 30 may comprise a transluminal section 85, a far side lateral projection 90 and a near side lateral projection 95, with the far side lateral projection 90 and the near side lateral projection 95 being held in opposition to one another so as to close down the lumen 60 of the vein 15. Such an arrangement may be provided by many different types of structures, e.g., such as the "double T-bar" structure shown in Figs. 25-27 where the transluminal section 85 of the occluder 30 is formed out of an elastic material which draws the two opposing T-bars 90, 95 of the occluder together so as to provide vessel occlusion. Still other arrangements for connecting and drawing together a far side lateral projection 90 and a near side lateral projection 95 will be apparent to those skilled in the art in view of the present disclosure. By way of further example, far side lateral projection 90 and near side lateral projection 95 may be connected together by a loop of suture, with the loop of suture being lockable in a reduced size configuration (i.e., so as to maintain occlusion) with a sliding locking knot.

Furthermore, multiple occluders 30 may be used on a single blood vessel or tissue to occlude the blood vessel more completely, or to occlude a blood vessel in multiple regions, or to attach a material (e.g., a drug or cellular delivery element) in multiple places to the blood vessel. The occluders may be coated with a drug-eluting compound, or the occluders may be electrically charged to enhance or prevent clotting or to deliver a desired compound or agent to the blood vessel, etc. If desired, the location of the occluding or attachment element may be precisely controlled to deliver the desired compound or agent at a specific anatomical location.

### Endoluminal Approach

In the endoluminal approach, the occluder 30 is delivered to the occlusion site by endoluminally advancing the occluder up the vein using a catheter, and then deploying the occluder in the vein, with the occluder acting to occlude the vein and thereby treat varicose veins. In this form, the occluder is preferably passed through one or more side walls of the vein so as to stabilize the occluder relative to the vein. In one form, the occluder is configured to occlude the vein by depositing a mass within the lumen of the vein so as to restrict blood flow through the lumen of the vein; and in another form, the occluder is configured to occlude the vein by compressing the vein and closing down its lumen.

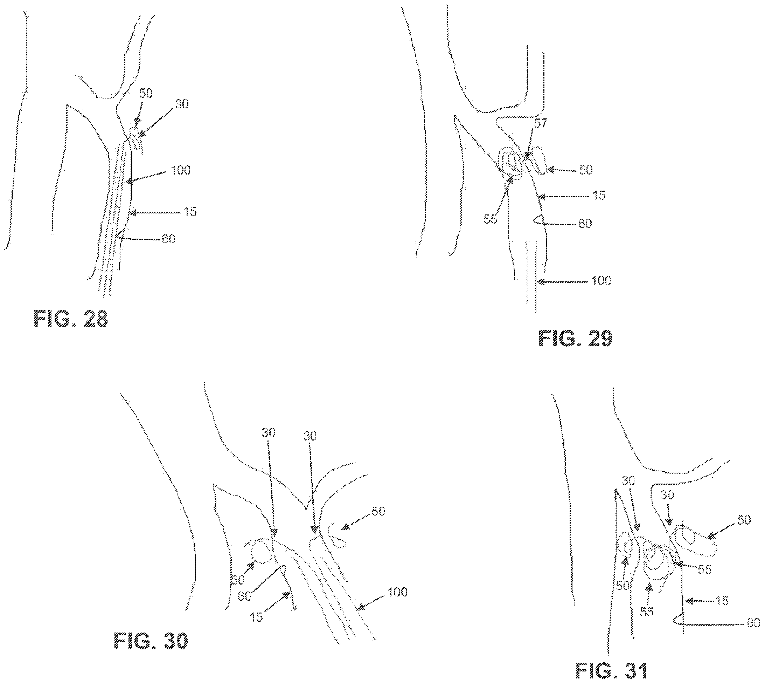

More particularly, and looking now at Figs. 28 and 29, a catheter 100 is used to endoluminally advance the occluder 30 up the interior of the vein 15 to a deployment site. Then one end of the occluder is passed through the side wall of the vein so as to deposit a coiled mass 50 of the occluder 30 outside the vein, and the remainder of the occluder is deposited as a coiled mass 55 within the lumen 60 of the vein, with a portion 57 of the occluder extending through the side wall of the vein so as to attach the occluder to the side wall of the vein and thereby stabilize the occluder relative to the vein. Thus, a coiled mass 55 of the occluder is deposited within the interior of the vein so as to restrict blood flow through the vein and thereby treat varicose veins.

Figs. 30 and 31 show how two separate occluders 30, each used in the manner shown in Figs. 28 and 29, can be used to increase the coiled mass of occluder contained within the lumen of the vein, whereby to increase the extent of occlusion of the lumen of the vein.

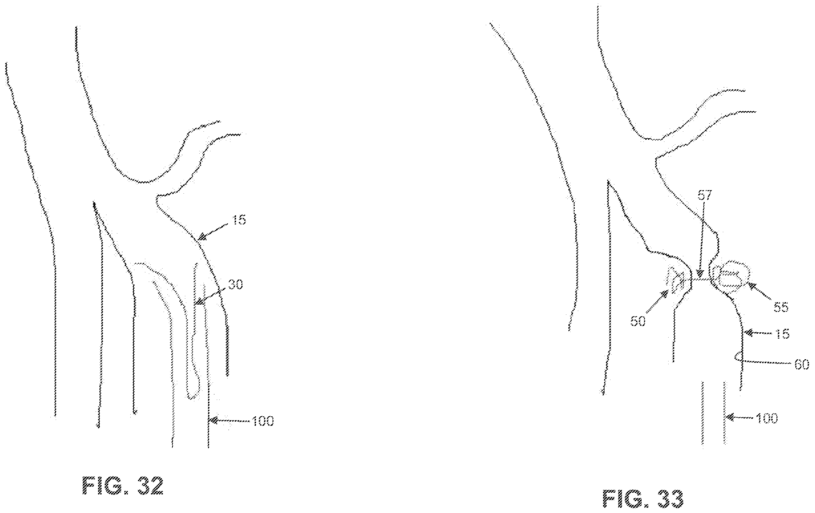

Figs. 32 and 33 show how an occluder 30 can be delivered endoluminally and used to compress the outer walls of the vein so as to occlude blood flow through the lumen of the vein. More particularly, the occluder 30 is advanced endoluminally through the vein to the deployment site, one end of the occluder is passed through one side wall of the vein so as to deposit a coiled mass 50 on one side of the vein and the other end of the occluder is passed through the other side wall of the vein so as to deposit another coiled mass 55 on the other side of the vein, with the two coiled masses being connected together by the intermediate portion 57 of the occluder and with the two coiled masses being drawn toward one another under the coiling force inherent in the elastic filament so as to apply compressive opposing forces on the two sides of the vein, whereby to compress the vein and close down its lumen.

### Occlusion In Combination With Phlebectomy

If desired, the described occluder can be used in conjunction with the removal of the large varicose veins (i.e., phlebectomy). The phlebectomy can be done at the same time as the occlusion of the vein or at another time. For this surgical procedure, minimal local anesthetic is needed.

### Occluding Tubular Structures For Purposes Other Than Treating Varicose Veins

It will be appreciated that the described occluder also be used to occlude tubular structures for purposes other than treating varicose veins. By way of example but not limitation, the described occluder can be used to occlude other vascular structures (e.g., to occlude arteries so as to control bleeding), or to occlude other tubular structures within the body (e.g., phallopian tubes, so as to induce infertility), etc.

### Drug/Cellular Delivery Applications

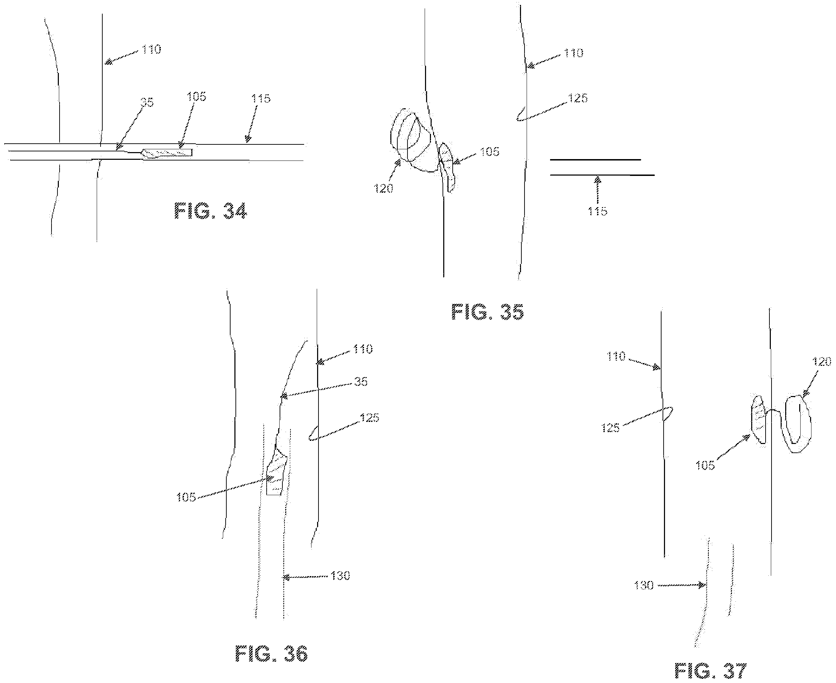

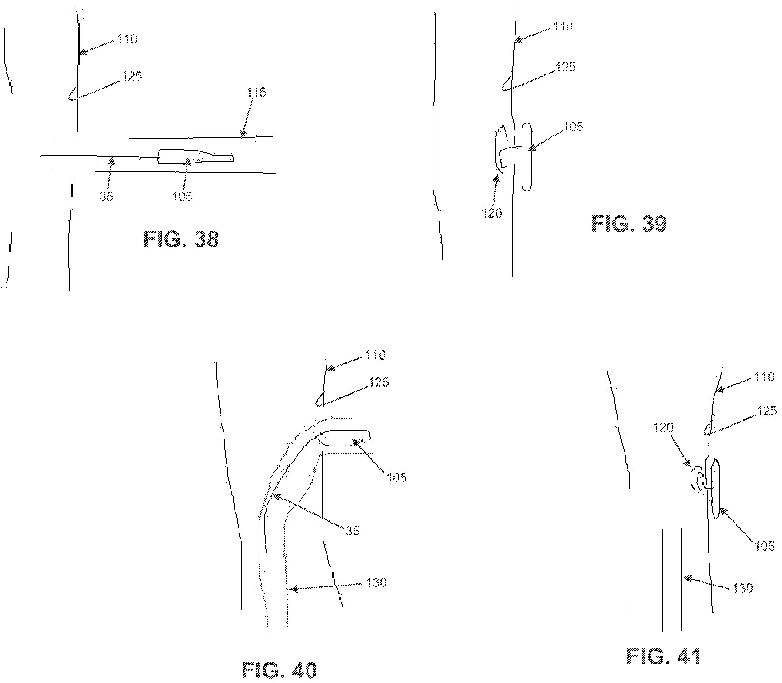

Furthermore, using the foregoing concept of minimally-invasive hollow tube penetration, and attachment and fixation of the device to the vessel wall, either percutaneously or endoluminally, the occluder 30 may be modified so as to allow drug/cellular delivery at fixed points within or adjacent to the vasculature or other hollow bodily structure. In this form, the device functions as a drug/cellular delivery stabilizer, and may or may not function as an occluder. See, for example, Figs. 34 and 35, where an elastic filament 35, having a drug/cellular delivery body 105 attached thereto, is advanced across a blood vessel 110 using a needle 115, with the distal end of the elastic filament forming a coiled mass 120 on the far side of the blood vessel and the drug/cellular delivery body 105 being securely disposed within the lumen 125 of the blood vessel. Figs. 36 and 37 show a similar arrangement where a catheter 130 is used to deliver the device endoluminally. Figs. 38 and 39 show another arrangement wherein the device is delivered percutaneously so that the coiled mass is disposed inside lumen 125 of the blood vessel and the drug/cellular delivery body 105 is disposed outside the blood vessel, and Figs. 40 and 41 show how the device is delivered endoluminally so that the coiled mass is disposed inside lumen 125 of the blood vessel and the drug/cellular delivery body 105 is disposed outside the blood vessel. These drug/cellular delivery devices may be passive or active polymers or silicon-based or micro- and nanotechnology devices, or matrices of materials, etc.

### Two-Part Occluder

Looking next at Fig. 42, there is shown a two-part occluder 200 formed in accordance with the present invention. Two-part occluder 200 generally comprises a distal implant 205 and a proximal implant 210.

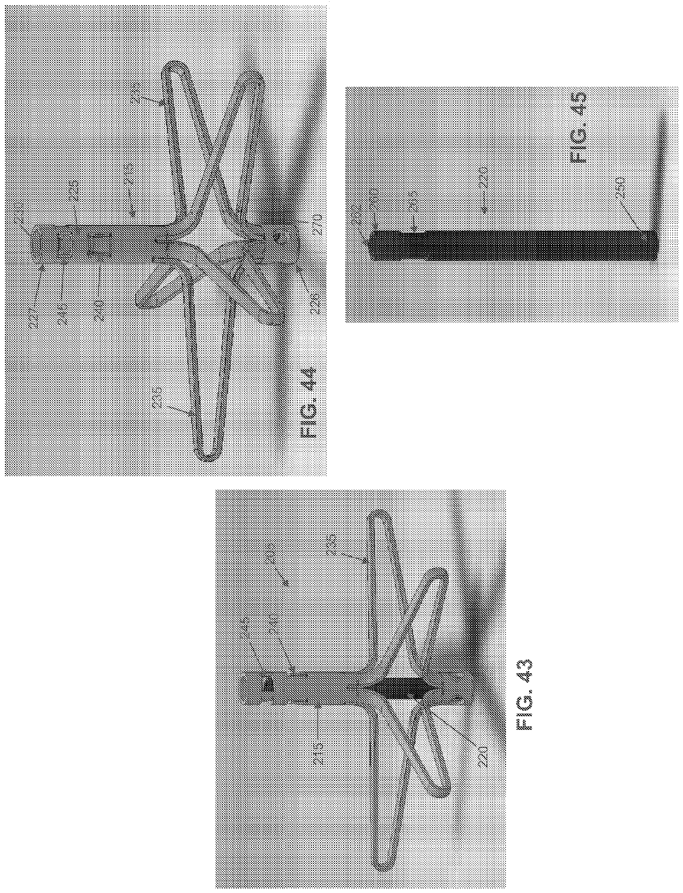

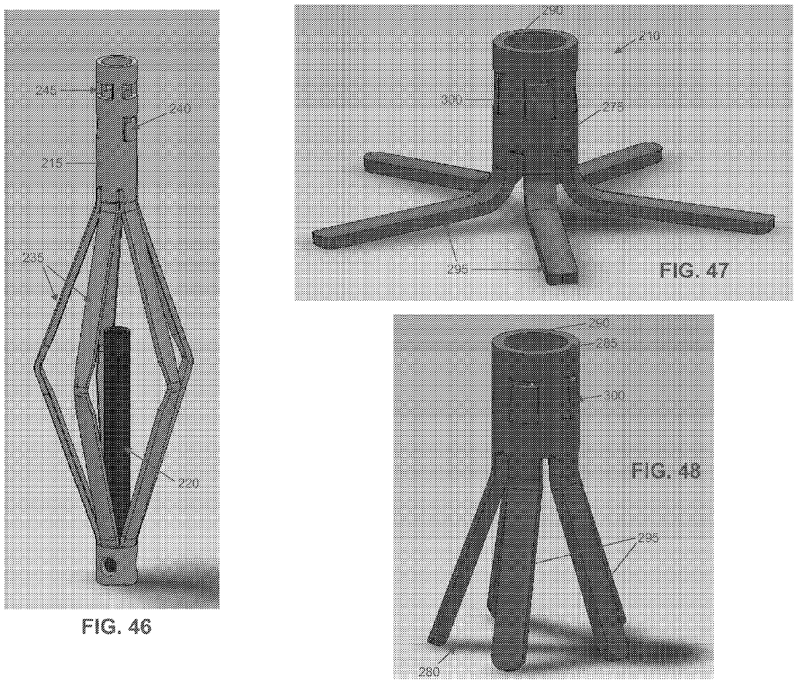

Distal implant 205 is shown in further detail in Figs. 43-46. Distal implant 205 comprises a distal implant body 215 and a distal implant locking tube 220. Distal implant body 215 comprises a tube 225 having a distal end 226, a proximal end 227, and a lumen 230 extending therebetween. Tube 225 is slit intermediate its length so as to define a plurality of legs 235. A set of inwardly-projecting tangs 240 are formed in tube 225 between legs 235 and proximal end 227. A set of windows 245 are formed in tube 225 between inwardly-projecting tangs 240 and proximal end 227. Distal implant body 215 is preferably formed out of an elastic material (e.g., a shape memory material having superelastic properties such as Nitinol) and constructed so that its legs 235 normally project laterally away from the longitudinal axis of tube 225 (e.g., in the manner shown in Figs. 43 and 44), however, due to the elastic nature of the material used to form distal implant body 215, legs 235 can be constrained inwardly (e.g., within the lumen of a delivery needle, as will hereinafter be discussed) so that distal implant body 215 can assume a substantially linear disposition. See, for example, Fig. 46, which shows legs 235 moved inwardly relative to the position shown in Figs. 43 and 44. However, when any such constraint is removed, the elastic nature of the material used to form distal implant body 215 causes legs 235 to return to the position shown in Figs. 43 and 44.

Distal implant locking tube 220 (Fig. 45) comprises a generally tubular structure having a distal end 250, a proximal end 260 and a lumen 262 extending therebetween. A set of windows 265 are formed in the distal implant locking tube 220, with windows 265 being disposed distal to proximal end 260.

Distal implant locking tube 220 is disposed within lumen 230 of distal implant body 215. When distal implant 205 is in its aforementioned substantially linear condition (i.e., with legs 235 restrained in an in-line condition), distal implant locking tube 220 terminates well short of tangs 240 of distal implant body 215, so that the proximal end 227 of distal implant body 215 can move longitudinally relative to distal end 226 of distal implant body 215. However, when the proximal end 227 of distal implant body 215 is moved distally a sufficient distance to allow full radial expansion of legs 235 (see Fig. 42), locking tangs 240 of distal implant body 215 will be received within windows 265 of distal implant locking tube 220, whereby to lock distal implant 205 in its radially-expanded condition (i.e., with legs 235 projecting laterally away from the longitudinal axis of tube 225, e.g., in the manner shown in Figs. 43 and 44). Spot welds applied via openings 270 formed in the distal end 226 of distal implant body 215 serve to lock distal implant locking tube 220 to distal implant body 215, whereby to form a singular structure (see Fig. 43 and 46).

Looking next at Figs. 47 and 48, proximal implant 210 comprises a tube 275 having a distal end 280, a proximal end 285, and a lumen 290 extending therebetween. Tube 275 is slit at its distal end so as to define a plurality of legs 295. A set of inwardly-projecting tangs 300 are formed in tube 275 between legs 295 and proximal end 285. Proximal implant 210 is preferably formed out of an elastic material (e.g., a shape memory material having superelastic properties such as Nitinol) and constructed so that its legs 295 normally project laterally away from the longitudinal axis of tube 275 (e.g., in the manner shown in Fig. 47), however, legs 295 can be constrained inwardly (e.g., within the lumen of a delivery tube, as will hereinafter be discussed) so that proximal implant 210 can assume a substantially linear disposition. See, for example, Fig. 48, which shows legs 295 moved inwardly relative to the position shown in Fig. 47. However, when any such constraint is removed, the elastic nature of the material used to form proximal implant 210 causes legs 295 to return to the position shown in Fig. 47.

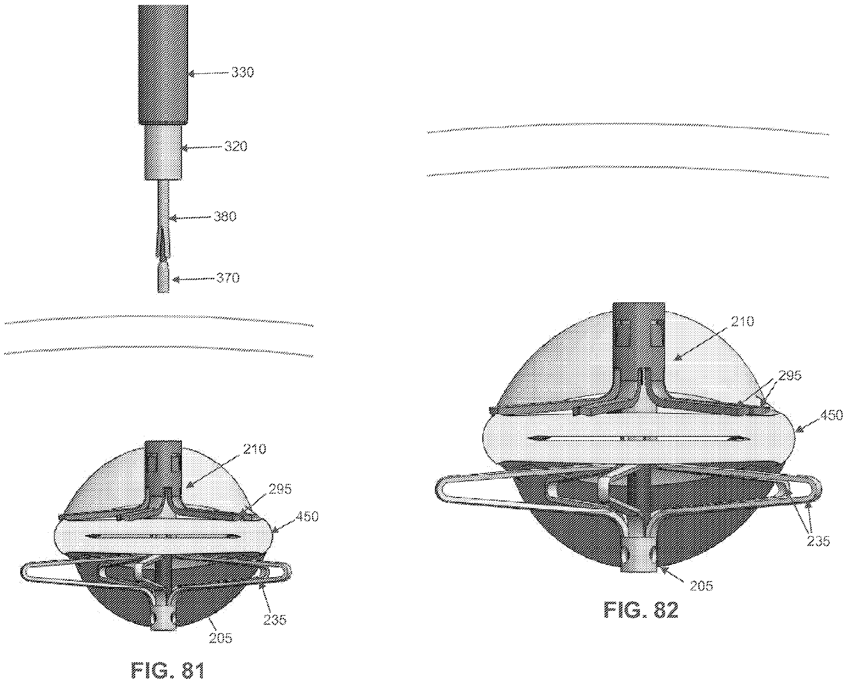

As will hereinafter be discussed, distal implant 205 and proximal implant 210 are configured and sized so that tube 225 of distal implant body 215 can be received in lumen 290 of proximal implant 210, with the expanded legs 235 of distal implant 205 opposing the expanded legs 295 of proximal implant 210 (see, for example, Fig. 82), whereby to impose a clamping action on the side wall of a blood vessel (e.g., vein) disposed therebetween and thereby occlude the blood vessel, as will hereinafter be discussed in further detail (or, as an alternative, the opposing expanded legs of the proximal and distal implants could interdigitate to impose the clamping action). Furthermore, distal implant 205 and proximal implant 210 are configured and sized so that they may be locked in this position, inasmuch as inwardly-projecting tangs 300 of proximal implant 210 will project into windows 245 of distal implant 205.

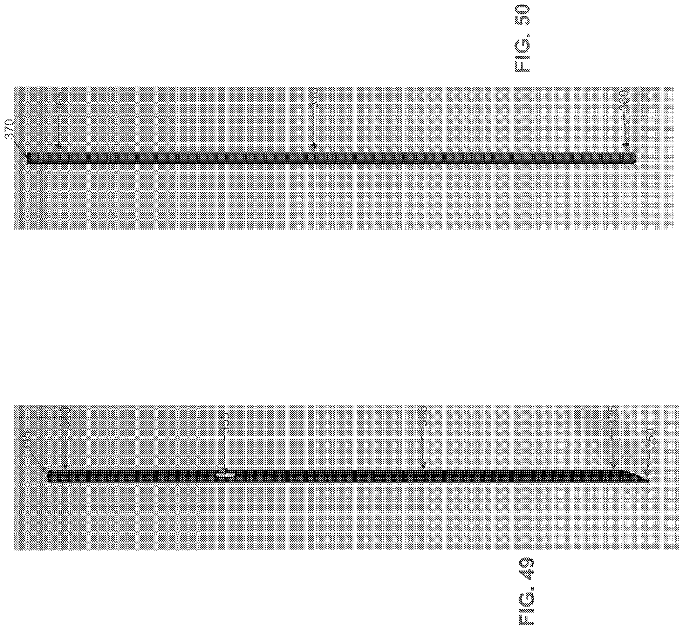

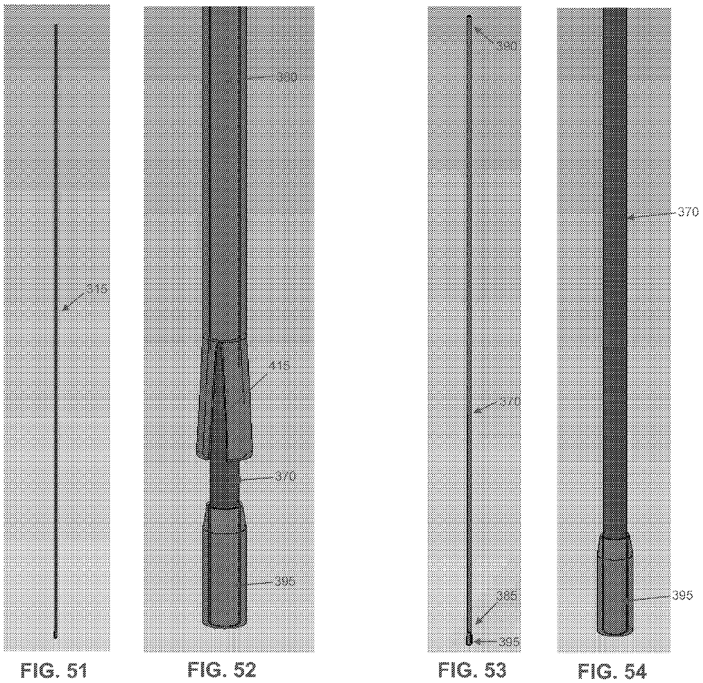

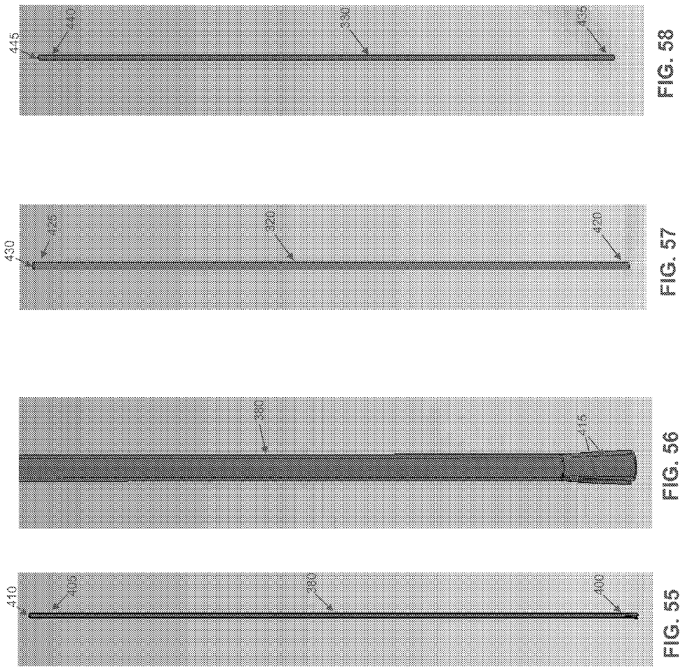

Two-part occluder 200 is intended to be deployed using associated installation apparatus. This associated installation apparatus preferably comprises a hollow needle 305 (Fig. 49) for penetrating tissue, a distal implant delivery tube 310 (Fig. 50) for delivering distal implant 205 through hollow needle 305 to the far side of the blood vessel which is to be occluded, a composite guidewire 315 (Figs. 51-56) for supplying support to various components during delivery and deployment, a push rod 320 (Fig. 57) for delivering various components over composite guidewire 315, and a proximal implant delivery tube 330 (Fig. 58) for delivering proximal implant 210 for mating with distal implant 205, as will hereinafter be discussed.

Hollow needle 305 (Fig. 49) comprises a distal end 335, a proximal end 340 and a lumen 345 extending therebetween. Distal end 335 terminates in a sharp point 350. In one preferred form of the invention, hollow needle 305 comprises a side port 355 which communicates with lumen 345.

Distal implant delivery tube 310 (Fig. 50) comprises a distal end 360, a proximal end 365 and a lumen 370 extending therebetween.

Composite guidewire 315 (Figs. 51-56) comprises a guidewire rod 370 and a guidewire sheath 380. Guidewire rod 370 comprises a distal end 385 and a proximal end 390. Distal end 385 terminates in an enlargement 395. Guidewire sheath 380 comprises a distal end 400, a proximal end 405 and a lumen 410 extending therebetween. The distal end 400 of guidewire sheath 380 comprises at least one, and preferably a plurality of, proximally-extending slits 415. Proximally-extending slits 415 open on the distal end of guidewire sheath 380 and allow the distal end of guidewire sheath 380 to radially expand somewhat. As will hereinafter be discussed, guidewire rod 370 and guidewire sheath 380 are configured and sized so that guidewire rod 370 can be received in lumen 410 of guidewire sheath 380. Furthermore, when guidewire rod 370 is forced proximally relative to guidewire sheath 380, the proximally-extending slits 415 in guidewire sheath 380 allow the distal end of the guidewire sheath 380 to expand somewhat so as to receive at least some of the enlargement 395 formed on the distal end of guidewire rod 370. As this occurs, the distal end of guidewire sheath 380 will expand radially.

Push rod 320 (Fig. 57) comprises a distal end 420, a proximal end 425 and a lumen 430 extending therebetween.

Proximal implant delivery tube 330 (Fig. 58) comprises a distal end 435, a proximal end 440 and a lumen 445 extending therebetween.

Two-part occluder 200 and its associated installation apparatus are preferably used as follows.

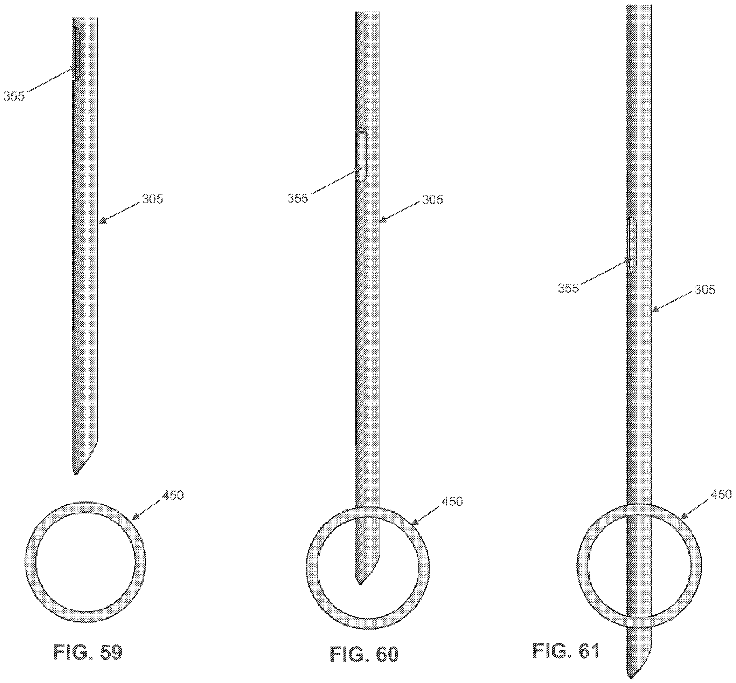

First, hollow needle 305 (carrying distal implant delivery tube 310 therein, which in turn contains the composite guidewire 315 therein, upon which is mounted distal implant 205) is passed through the skin of the patient, through intervening tissue, and across the blood vessel (e.g., vein 450) which is to be occluded. See Figs. 59-61. As this is done, any blood flowing out side port 355 can be monitored - excessive or pulsatile blood flow can indicate that hollow needle has accidentally struck an artery.

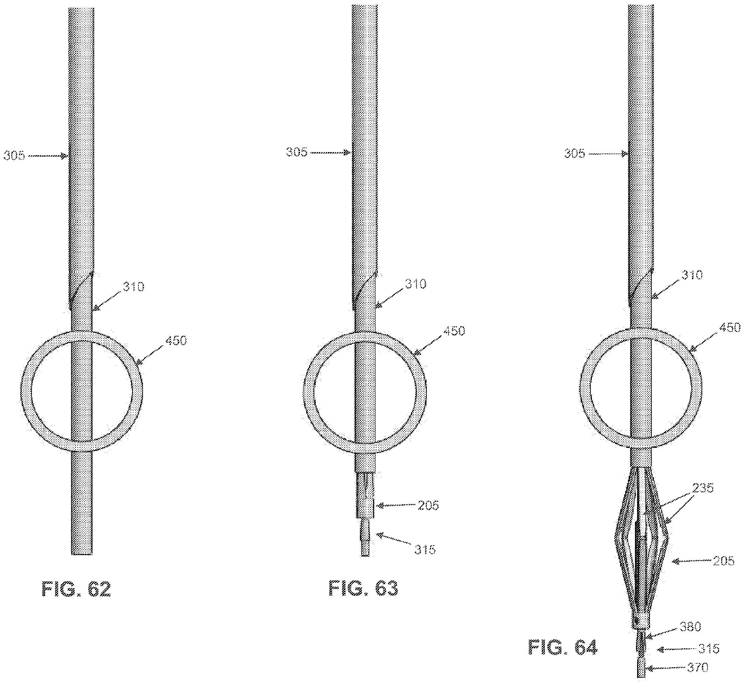

Next, hollow needle 305 is retracted, leaving distal implant delivery tube 310 extending across the blood vessel. See Fig. 62.

Then distal implant delivery tube 310 is retracted somewhat so as to expose the distal ends of composite guidewire 315 and distal implant 205. See Fig. 63.

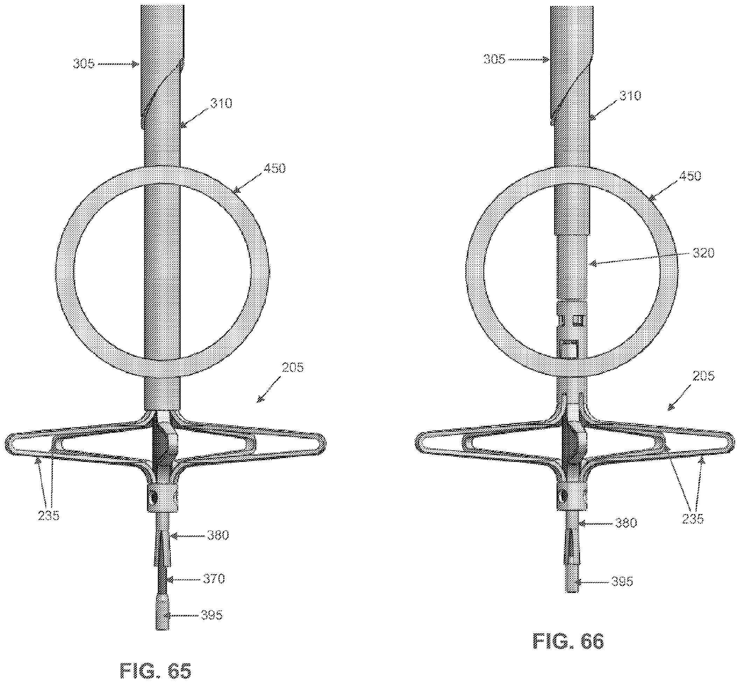

Next, composite guidewire 315, push rod 320 and distal implant 205 are all moved distally, so as to advance the distal ends of composite guidewire 315 and the distal implant 205 out of the distal end of distal implant delivery tube 310. As this occurs, legs 235 of distal implant 205 are released from the constraint of distal implant delivery tube 310 and expand radially. See Figs. 64 and 65.

Then, with push rod 320 being held in place against the proximal end of distal implant 205, composite guidewire 315 is pulled proximally so as to bring the distal end of distal implant 205 toward the proximal end of distal implant 205, whereby to cause locking tangs 240 of distal implant body 215 to enter windows 265 of distal implant locking tube 220, whereby to lock legs 235 in their radially-expanded condition (see Fig. 66).

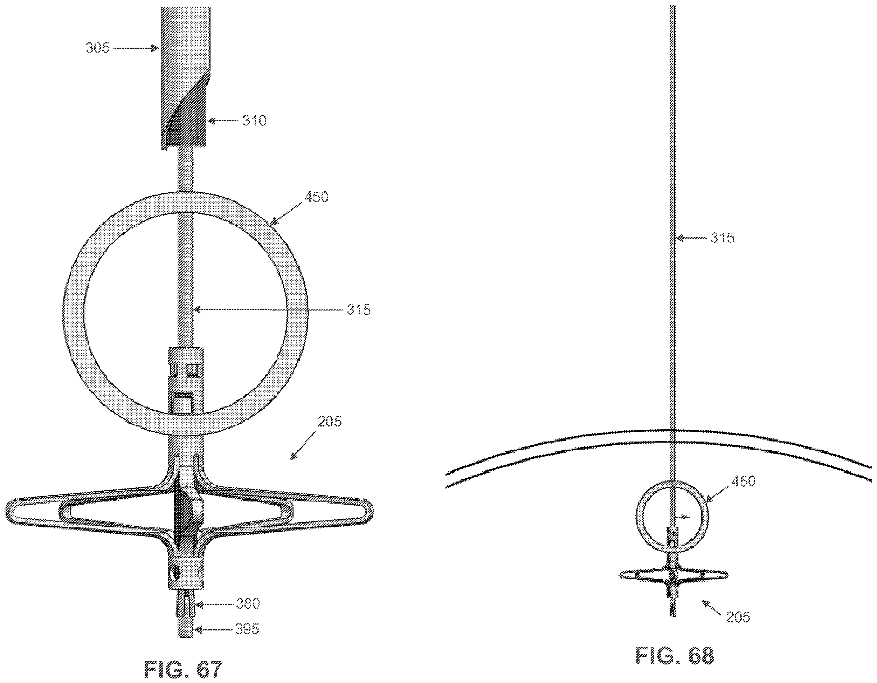

At this point, hollow needle 305, distal implant delivery tube 310 and push rod 320 may be removed (Fig. 67), leaving distal implant 205 mounted on composite guidewire 315, with the legs 235 fully deployed on the far side of the blood vessel and the proximal end of distal implant 205 extending into the interior of the blood vessel (Fig. 68).

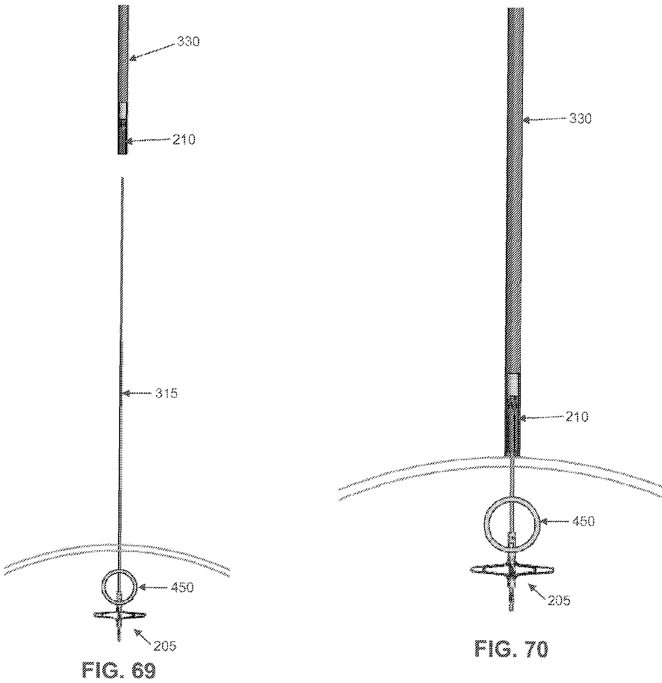

Next, proximal implant delivery tube 330 (carrying proximal implant 210 therein) is advanced down composite guidewire 315, until the distal end of proximal implant delivery tube 330 sits just proximal to the blood vessel (Figs. 69-72).

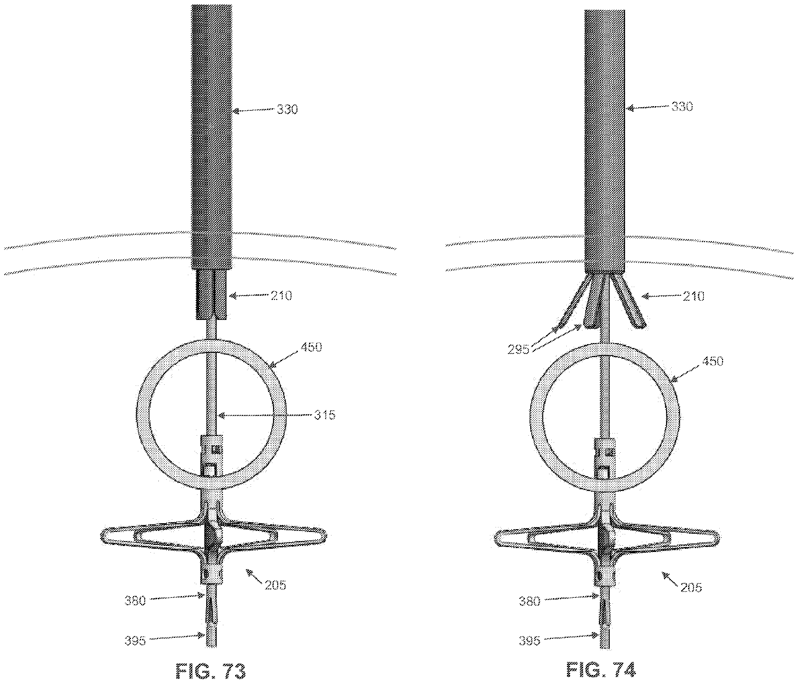

Then push rod 320 is used to advance the distal end of proximal implant 210 out of the distal end of proximal implant delivery tube 330. As this occurs, legs 295 are released from the constraint of proximal implant delivery tube 330 and open radially. See Figs. 73-76.

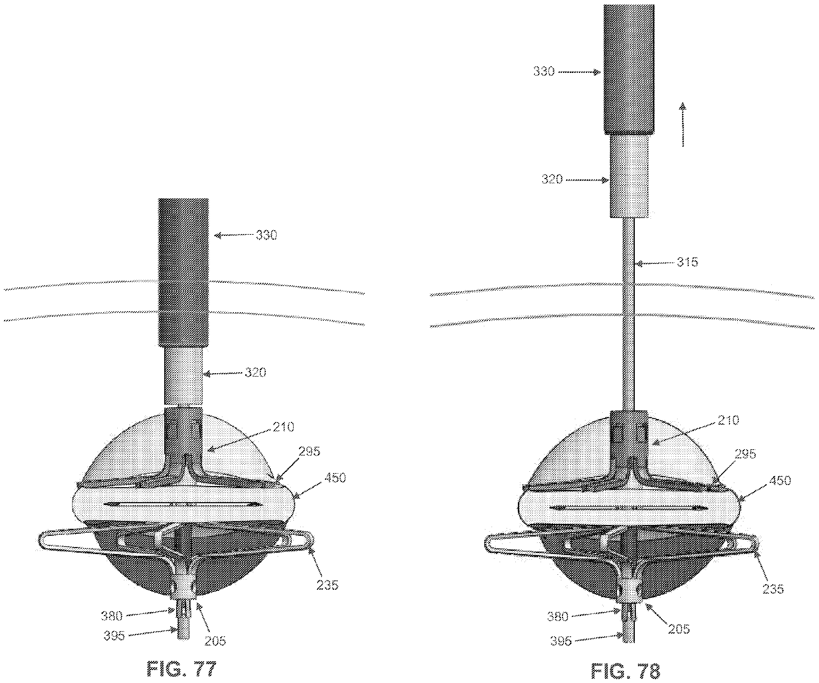

Next, using push rod 320, proximal implant 210 is pushed distally as distal implant 205 is pulled proximally using composite guidewire 315. More particularly, guidewire rod 370 is pulled proximally, which causes enlargement 395 on the distal end of guidewire rod 370 to expand guidewire sheath 380 to a size larger than lumen 262 in distal implant locking tube 220, which causes guidewire sheath 380 to move proximally, which causes proximal movement of distal implant 205. As distal implant 205 and proximal implant 210 move together, their legs 235, 295 compress the blood vessel, thereby occluding the blood vessel. Distal implant 205 and proximal implant 210 continue moving together until inwardly-projecting tangs 300 of proximal implant 210 enter windows 245 of distal implant 205, thereby locking the two members into position relative to one another. See Fig. 77.

At this point push rod 320 and proximal implant delivery tube 330 are removed. See Fig. 78.

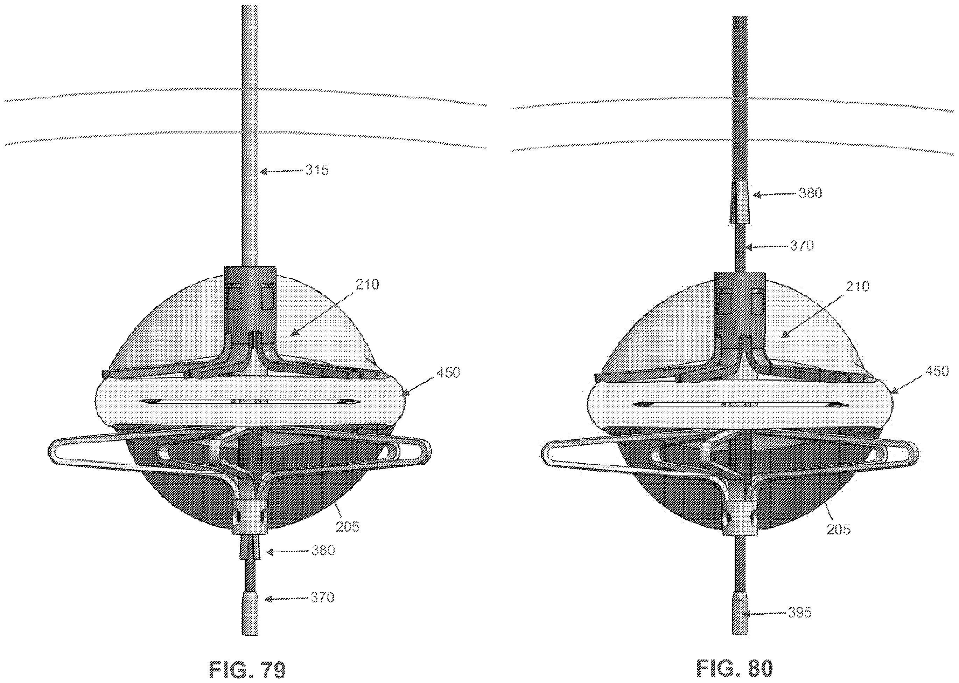

Next, composite guidewire 315 is removed. This is done by first advancing guidewire rod 370 distally (Fig. 79), which allows the distal end of guidewire sheath 380 to relax inwardly, thereby reducing its outer diameter to a size smaller than lumen 262 in distal implant locking tube 220. As a result, guidewire sheath 380 can then be withdrawn proximally through the interior of two-part occluder 200. See Fig. 80. Then guidewire rod 370 can be withdrawn proximally through the interior of two-part occluder 200. See Fig. 81.

The foregoing procedure leaves two-part occluder 200 locked in position across the blood vessel, with the opposing legs 235, 295 compressing the blood vessel, whereby to occlude the blood vessel.

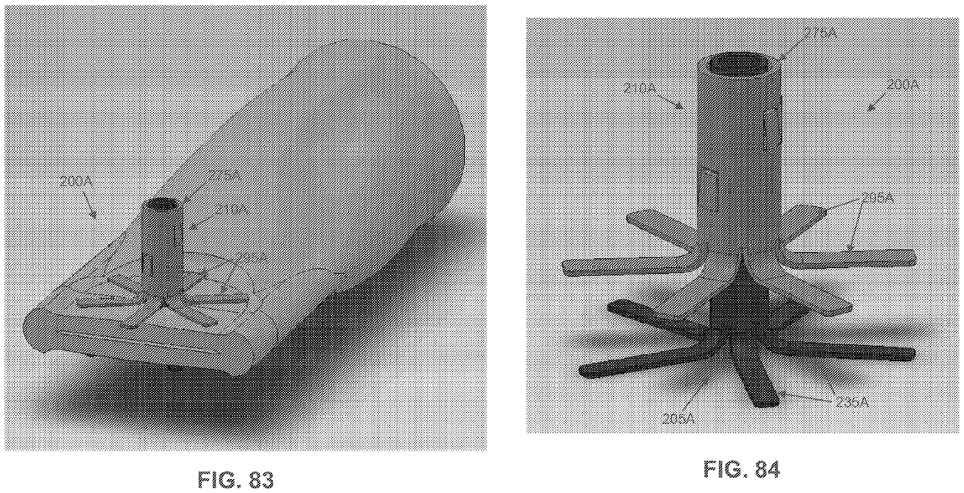

Figs. 83-86 illustrate another two-part occluder 200A having a distal implant 205A and a proximal implant 210A. Two-part occluder 200A is generally similar to the aforementioned two-part occluder 200, except that distal implant 205A utilizes a unibody construction.

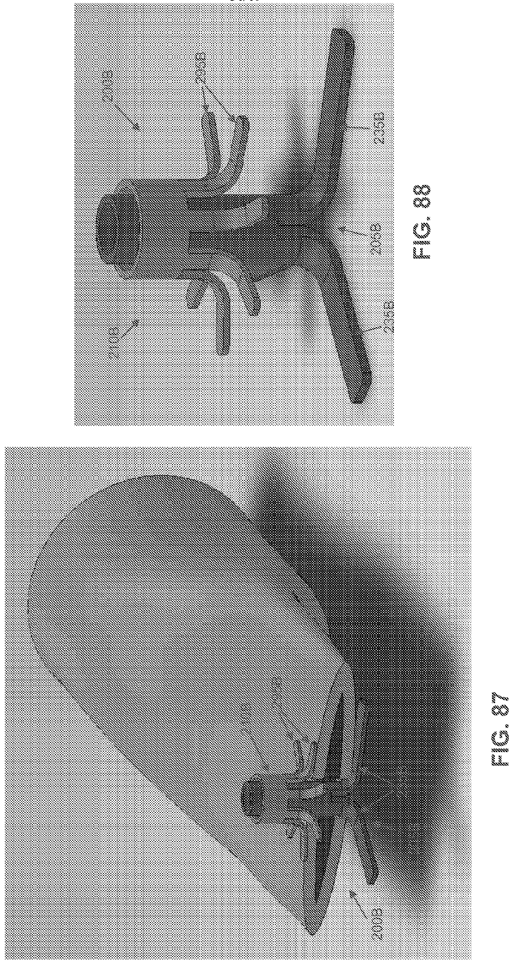

Figs. 87-90 illustrate another two-part occluder 200B. Two-part occluder 200B is generally similar to the aforementioned two-part occluder 200A, except that distal implant 205B utilizes a friction fit to lock distal implant 205B to proximal implant 210B.

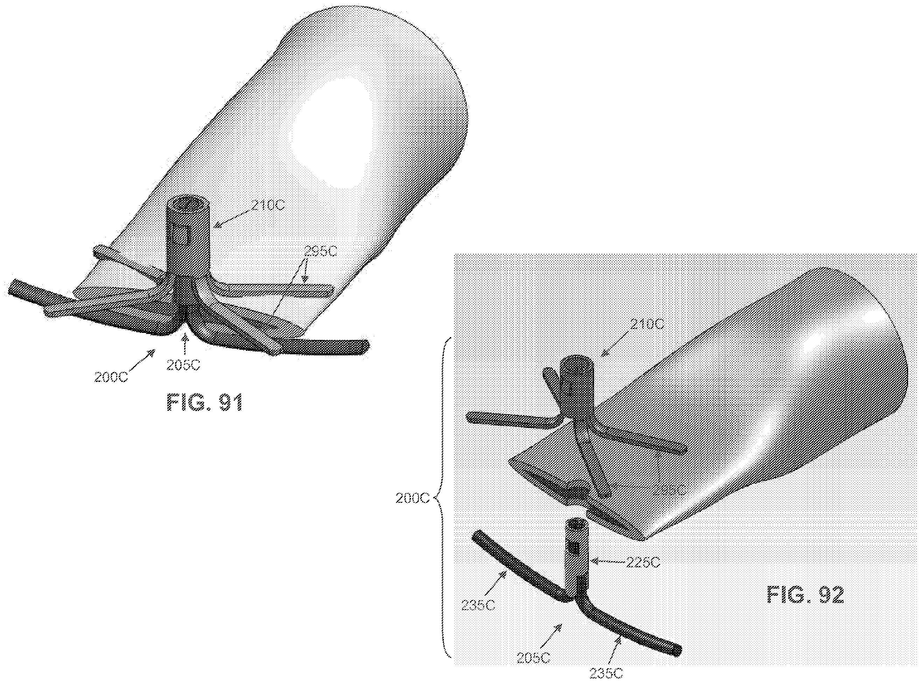

Figs. 91-94 illustrate another two-part occluder 200C having a distal implant 205C and a proximal implant 210C. Two-part occluder 200C is generally similar to the aforementioned two-part occluder 200, except that distal implant 205C comprises a tube 225C which receives and secures the proximal ends of legs 235C. Legs 235C are preferably elongated elements (e.g., bent wires) formed out of a superelastic shape memory material so as to provide the legs 235C with the desired degree of elasticity.

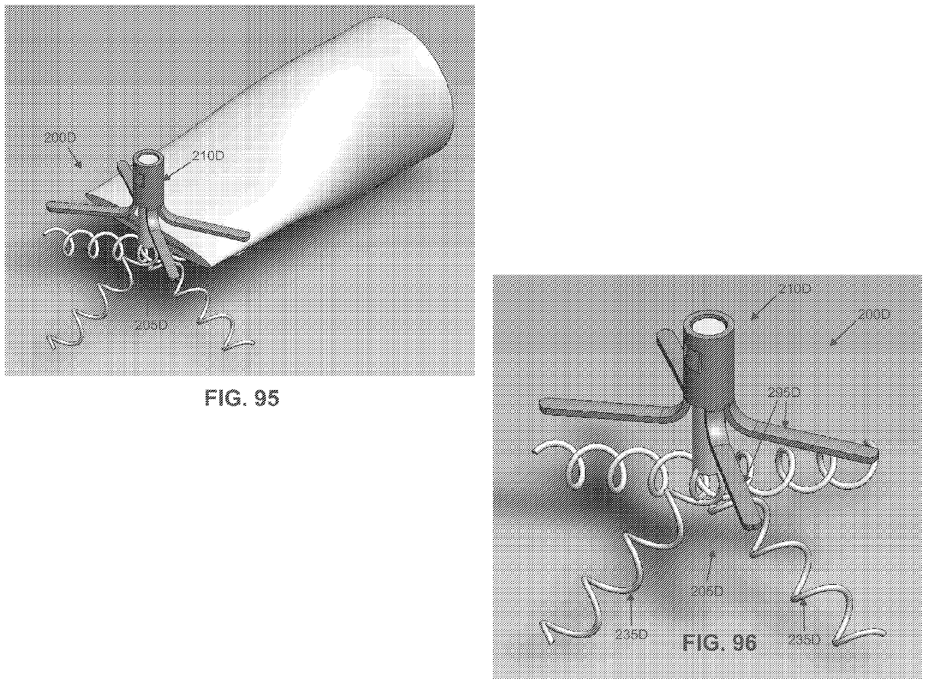

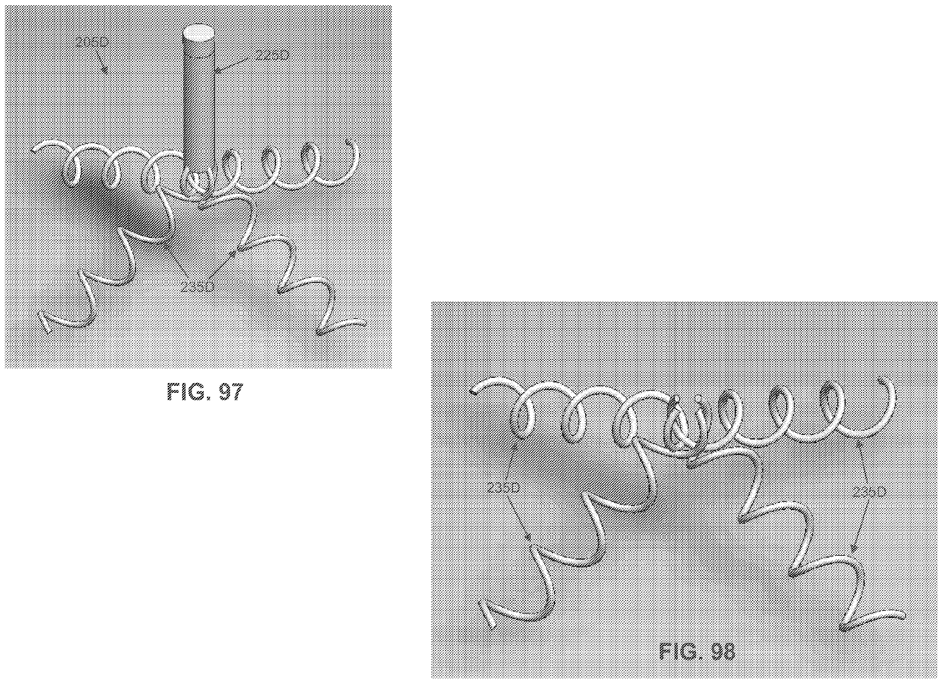

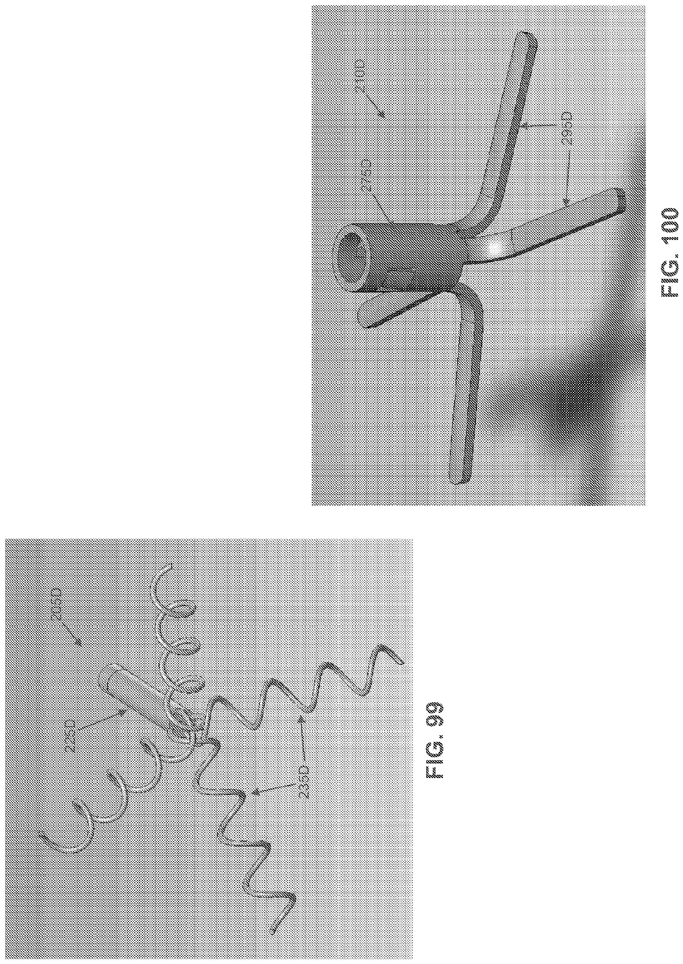

Figs. 95-100 illustrate another two-part occluder 200D not according to the invention, having a distal implant 205D and a proximal implant 210D. Two-part occluder 200D is generally similar to the aforementioned two-part occluder 200, except that distal implant 205D comprises a tube or rod 225D which receives and secures the proximal ends of legs 235D. Legs 235D are preferably coils formed out of a superelastic shape memory material so as to provide the legs 235D with the desired degree of elasticity.

In the foregoing disclosure, there is a disclosed a composite guidewire 315 for use in delivering distal implant 205 and proximal implant 210 to the anatomy. As noted above, composite guidewire 315 is formed from two parts, i.e., a guidewire rod 370 and a guidewire sheath 380. By providing composite guidewire 315 with this two-part construction, composite guidewire 315 can have its distal diameter enlarged or reduced as desired so as to permit composite guidewire 315 to bind to distal implant 205, or be separable from the distal implant 205, respectively. However, if desired, composite guidewire 315 can be replaced by an alternative guidewire which includes a mechanism for releasably binding the alternative guidewire to distal implant 205. By way of example but not limitation, such an alternative guidewire may include screw threads, and distal implant 205 may include a screw recess, so that the alternative guidewire can be selectively secured to, or released from, the distal implant 205, i.e., by a screwing action.

### Modifications Of The Preferred Embodiments

It should be understood that many additional changes in the details, materials (e.g., shape memory polymers that are permanent or that dissolve over time, or carbon nanotube based), steps and arrangements of parts, which have been herein described and illustrated in order to explain the present invention, may be made by those skilled in the art while still remaining within the scope of the invention.

## Claims

1. An apparatus for securing tissue layers together comprising:
a distal implant (205) comprising a tubular distal body (225) having a first locking element (245) and a plurality of distal legs (235) which may be resiliently deformed to a diametrically-reduced delivery configuration in which the distal implant (205) can be contained within a delivery tube (310) to be passed through at least two tissue layers, the legs (235) being diametrically self-expandable when released from a delivery tube (310) to a deployed configuration in which the legs (235) extend radially of the distal body, the legs (235) being defined by a plurality of longitudinally extending slits formed partially along the tubular distal body; and
a tubular proximal implant (210), separate from but connectible to the distal implant (205), the proximal implant (210) comprising a proximal tubular body (275) having a second locking element (300) and a plurality of proximal legs (295) that may be resiliently deformed to a diametrically-reduced delivery configuration and are diametrically self-expandable to a deployed configuration in which the legs extend radially of the proximal tubular body;
**characterized by** one of the distal implant (205) and the proximal implant (210) being receivable within the other to connect the first locking element (245) and the second locking element, thereby locking the distal implant and the proximal implant together whereby tissue layers can be engaged between the proximal and distal legs , the deployed legs and the first and second locking elements being configured and positioned so that, when the implants are locked together, the legs of the proximal implant are interdigitated with the legs of the distal implant.

2. The apparatus according to claim 1, wherein the distal implant is received within the proximal implant, further comprising:
the slits that define the legs of the distal implant (205) are formed along the distal body between the ends of the distal body; and
a locking tube (220) attached to and extending proximally from the distal end (226) of the distal tube and being receivable in the proximal end (227) of the distal tube (225) when the legs (235) are in a radially extended position, the locking tube (220) and the proximal portion of the distal tube (225) having cooperative locking elements (240, 265) to secure the distal implant (205) in a configuration in which the legs (235) extend radially.

3. The apparatus according to claim 1 wherein legs (225) of the proximal implant (210), when deployed, are rotationally offset with legs (235) of the deployed distal implant. (205).

4. The apparatus according to claim 1 wherein the implants (210) are formed from a shape memory material having superelastic characteristics.

5. The apparatus according to claim 1 further comprising a mechanism (315) for maintaining the position of the distal implant (205) after ejection out of the delivery tube; thereby to enable the delivery tube to be withdrawn proximally to deploy the distal implant (205) distally of the layers.

6. The apparatus according to claim 5 wherein the mechanism comprises an elongate guidewire (380) configured to fit axially through a lumen in the distal body, the guidewire having a selectively expandable locking region (415) configured to interfere with the lumen of the distal body lumen to maintain the position of the distal implant (205), the locking region being selectively configurable to a low profile to release the distal implant (205) and enable removal of the elongate guidewire through the lumen of the distal body,

7. The apparatus according to claim 5 further comprising a push rod (320) engageable with the proximal end of the proximal implant to maintain the position of the proximal implant proximally of the tissue layers.

8. The apparatus according to claim 7 wherein the guidewire and the push rod can be manipulated to draw the deployed proximal and distal implants (205) toward each other to lock them together.

9. The apparatus according to claim 1, wherein the distal implant is received within the proximal implant, further comprising:
the distal implant (205) having a locking tube (220) attached to and extending proximally from the distal implant (205), the distal implant (205) having a proximal tubular portion receptive to the locking tube (220);
the proximal tubular portion of the distal implant (220) having a third locking element (240) and the locking tube having a fourth locking element (265), the distal implant (205) being axially compressible to cause the distal legs (235) to deploy and to advance the proximal tubular portion of the distal implant (220) over the proximal end of the locking tube (220) and to cause the third and fourth locking elements to engage each other to lock the distal implant (205) with its legs (235) in a deployed configuration.

## Patentansprüche

1. Vorrichtung zum Befestigen von Gewebeschichten aneinander, die Folgendes umfasst:
ein distales Implantat (205), das einen rohrförmigen distalen Körper (225), der ein erstes Arretierelement (245) aufweist, und eine Vielzahl von distalen Beinen (235) umfasst, die elastisch auf eine durchmesserreduzierte Zufuhrkonfiguration verformt werden kann, in der das distale Implantat (205) innerhalb eines Zufuhrschlauchs (310) enthalten sein kann, um durch zumindest zwei Gewebeschichten geführt zu werden, wobei die Beine (235), wenn sie aus einem Zufuhrschlauch (310) freigegeben werden, ihren Durchmesser betreffend in eine eingesetzte Konfiguration selbstausfahrbar sind, in der sich die Beine (235) von dem distalen Körper radial erstrecken, wobei die Beine (235) durch eine Vielzahl von sich in Längsrichtung erstreckenden Schlitzen entlang des rohrförmigen distalen Körpers definiert sind; und
ein rohrförmiges proximales Implantat (210), das von dem distalen Implantat (205) getrennt aber mit diesem verbindbar ist, wobei das proximale Implantat (210) einen proximalen rohrförmigen Körper (275), der ein zweites Arretierelement (300) aufweist, und eine Vielzahl von proximalen Beinen (295) umfasst, die elastisch auf eine durchmesserreduzierte Zufuhrkonfiguration verformt werden können und ihren Durchmesser betreffend in eine eingesetzte Konfiguration selbstausfahrbar sind, in der sich die Beine von dem proximalen rohrförmigen Körper radial erstrecken;
**dadurch gekennzeichnet, dass** eines aus dem distalen Implantat (205) und dem proximalen Implantat (210)
innerhalb des anderen aufnehmbar ist, um das erste Arretierelement (245) und das zweite Arretierelement zu verbinden, wodurch das distale Implantat und das proximale Implantat miteinander arretiert werden, wodurch Gewebeschichten zwischen den proximalen und den distalen Beinen in Eingriff geraten können, wobei die eingesetzten Beine und das erste und das zweite Arretierelement so ausgelegt und positioniert sind, dass, wenn die Implantate miteinander arretiert sind, die Beine des proximalen Implantats mit den Beinen des distalen Implantats verschränkt sind.

2. Vorrichtung nach Anspruch 1, wobei das distale Implantat innerhalb des proximalen Implantats aufgenommen ist, wobei die Vorrichtung ferner Folgendes umfasst:
die Schlitze, die die Beine des distalen Implantats (205) bilden, sind entlang des distalen Körpers zwischen den Enden des distalen Körpers ausgebildet; und
ein Arretierrohr (220), das an dem distalen Ende (226) des distalen Rohrs angebracht und sich ausgehend von diesem proximal erstreckt und in dem proximalen Ende (227) des distalen Rohrs (225) aufnehmbar ist, wenn sich die Beine (235) in einer radial ausgefahrenen Position befinden, wobei das Arretierrohr (220) und der proximale Abschnitt des distalen Rohrs (225) zusammenwirkende Arretierelemente (240, 265) aufweisen, um das distale Implantat (205) in einer Konfiguration zu befestigen, in der sich die Beine (235) radial erstrecken.

3. Vorrichtung nach Anspruch 1, wobei Beine (225) des proximalen Implantats (210), wenn es eingesetzt wird, gegenüber Beinen (235) des eingesetzten distalen Implantats (205) rotationsmäßig versetzt sind.

4. Vorrichtung nach Anspruch 1, wobei die Implantate (210) aus einem Form-Memory-Material ausgebildet sind, das superelastische Eigenschaften aufweist.

5. Vorrichtung nach Anspruch 1, die ferner einen Mechanismus (315) zum Aufrechterhalten der Position des distalen Implantats (205) nach Ausstoßen aus dem Zufuhrschlauch umfasst, wodurch ermöglicht wird, dass der Zufuhrschlauch proximal zurückgezogen wird, um das distale Implantat (205) distal zu den Schichten einzusetzen.

6. Vorrichtung nach Anspruch 5, wobei der Mechanismus einen länglichen Führungsdraht (380) umfasst, der ausgelegt ist, um axial durch ein Lumen in dem distalen Körper zu passen, wobei der Führungsdraht einen selektiv erweiterbaren Arretierbereich (415) aufweist, der ausgelegt ist, um mit dem Lumen des distalen Körpers zusammenzuwirken, um die Position des distalen Implantats (205) beizubehalten, wobei der Arretierbereich auf ein kleines Profil selektiv konfigurierbar ist, um das distale Implantat (205) freizugeben und das Entfernen des länglichen Führungsdrahts durch das Lumen des distalen Körpers zu ermöglichen.

7. Vorrichtung nach Anspruch 5, die ferner eine Schubstange (320) umfasst, die mit dem proximalen Ende des proximalen Implantats in Eingriff bringbar ist, um die Position des proximalen Implantats proximal zu den Gewebeschichten beizubehalten.

8. Vorrichtung nach Anspruch 7, wobei der Führungsdraht und die Schubstange genutzt werden können, um das eingesetzte proximale und distale Implantat (205) zusammenzuziehen, um sie aneinander zu arretieren.

9. Vorrichtung nach Anspruch 1, wobei das distale Implantat innerhalb des proximalen Implantats aufgenommen ist, wobei die Vorrichtung ferner Folgendes umfasst:
das distale Implantat (205), das ein Arretierrohr (220) aufweist, das an dem distalen Implantat (205) angebracht ist und sich proximal davon erstreckt, wobei das distale Implantat (205) einen proximalen rohrförmigen Abschnitt aufweist, der das Arretierrohr (220) aufnehmen kann;
wobei der proximale rohrförmige Abschnitt des distalen Implantats (220) ein drittes Arretierelement (240) aufweist und das Arretierrohr ein viertes Arretierelement (265) aufweist, wobei das distale Implantat (205) axial zusammendrückbar ist, um zu bewirken, dass die distalen Beine (235) eingesetzt werden und der proximale rohrförmige Abschnitt des distalen Implantats (220) über das proximale Ende des Arretierrohrs (220) hinaus bewegt wird und um zu bewirken, dass das dritte und das vierte Arretierelement miteinander in Eingriff geraten, um das distale Implantat (205) mit seinen Beinen (235) in einer eingesetzten Konfiguration zu arretieren.

## Revendications

1. Appareil pour fixer des couches de tissu ensemble, comprenant :
un implant distal (205) comprenant un corps distal tubulaire (225) présentant un premier élément de verrouillage (245) et une pluralité de pattes distales (235) qui peuvent être déformées de manière élastique jusqu'à une configuration d'administration diamétralement réduite dans laquelle l'implant distal (205) peut être contenu à l'intérieur d'un tube d'administration (310) pour être passé à travers au moins deux couches de tissu, les pattes (235) étant diamétralement auto-expansibles lorsqu'elles sont libérées du tube d'administration (310) jusqu'à une configuration déployée dans laquelle les pattes (235) s'étendent radialement par rapport au corps distal, les pattes (235) étant définies par une pluralité de fentes s'étendant longitudinalement formées partiellement le long du corps distal tubulaire ; et
un implant proximal tubulaire (210), séparé de l'implant distal (205) mais pouvant être connecté à celui-ci, l'implant proximal (210) comprenant un corps tubulaire proximal (275) présentant un deuxième élément de verrouillage (300) et une pluralité de pattes proximales (295) qui peuvent être déformées de manière élastique jusqu'à une configuration d'administration diamétralement réduite et sont diamétralement auto-expansibles jusqu'à une configuration déployée dans laquelle les pattes s'étendent radialement par rapport au corps tubulaire proximal ;
**caractérisé en ce que** l'un de l'implant distal (205) et de l'implant proximal (210) peut être reçu dans l'autre pour connecter le premier élément de verrouillage (245) et le deuxième élément de verrouillage, en verrouillant ainsi l'implant distal et l'implant proximal ensemble, moyennant quoi des couches de tissu peuvent être mises en prises entre les jambes proximales et distales, les jambes déployées et les premier et deuxième éléments de verrouillage étant configurés et positionnés de telle sorte que lorsque les implants sont verrouillés ensemble, les pattes de l'implant proximal sont interdigitées avec les pattes de l'implant distal.

2. Appareil selon la revendication 1, dans lequel l'implant distal est reçu à l'intérieur de l'implant proximal, comprenant en outre :
les fentes qui définissent les pattes de l'implant distal (205) sont formées le long du corps distal entre les extrémités du corps distal ; et
un tube de verrouillage (220) fixé à et s'étendant proximalement depuis l'extrémité distale (226) du tube distal et pouvant être reçu dans l'extrémité proximale (227) du tube distal (225) lorsque les pattes (235) sont dans une position radialement étendue, le tube de verrouillage (220) et la partie proximale du tube distal (225) présentant des éléments de verrouillage coopératifs (240, 265) pour fixer l'implant distal (205) dans une configuration dans laquelle les pattes (235) s'étendent radialement.

3. Appareil selon la revendication 1, dans lequel les pattes (225) de l'implant proximal (210), lorsqu'elles sont déployées, sont décalées en rotation par rapport aux pattes (235) de l'implant distal déployé (205).

4. Appareil selon la revendication 1, dans lequel les implants (210) sont formés à partir d'un matériau à mémoire de forme présentant des caractéristiques superélastiques.

5. Appareil selon la revendication 1, comprenant en outre un mécanisme (315) pour maintenir la position de l'implant distal (205) après une éjection hors du tube d'administration, en permettant ainsi au tube d'administration d'être retiré de manière proximale pour déployer l'implant distal (205) de manière distale par rapport aux couches.

6. Appareil selon la revendication 5, dans lequel le mécanisme comprend un fil de guidage allongé (380) configuré pour s'adapter axialement à travers une lumière dans le corps distal, le fil de guidage présentant une région de verrouillage expansible sélectivement (415) configurée pour interférer avec la lumière de la lumière de corps distal afin de maintenir la position de l'implant distal (205), la région de verrouillage étant configurable sélectivement à un profil bas pour libérer l'implant distal (205) et permettre un retrait du fil de guidage allongé à travers la lumière du corps distal.

7. Appareil selon la revendication 5, comprenant en outre une tige de poussée (320) pouvant être mise en prise avec l'extrémité proximale de l'implant proximal afin de maintenir la position de l'implant proximal de manière proximale par rapport aux couches de tissu.

8. Appareil selon la revendication 7, dans lequel le fil de guidage et la tige de poussée peuvent être manipulés pour tirer les implants proximal et distal déployés (205) l'un vers l'autre afin de les verrouiller ensemble.

9. Appareil selon la revendication 1, dans lequel l'implant distal est reçu à l'intérieur de l'implant proximal, comprenant en outre :
l'implant distal (205) présentant un tube de verrouillage (220) attaché à et s'étendant de manière proximale à partir de l'implant distal (205), l'implant distal (205) présentant une partie tubulaire proximale réceptive au tube de verrouillage (220) ;
la partie tubulaire proximale de l'implant distal (220) présentant un troisième élément de verrouillage (240) et le tube de verrouillage présentant un quatrième élément de verrouillage (265), l'implant distal (205) étant compressible axialement pour amener les pattes distales (235) à se déployer et pour faire avancer la partie tubulaire proximale de l'implant distal (220) sur l'extrémité proximale du tube de verrouillage (220) et pour amener les troisième et quatrième éléments de verrouillage à venir en prise l'un avec l'autre afin de verrouiller l'implant distal (205) avec ses pattes (235) dans une configuration déployée.
